(19) Europäisches Patentamt
European Patent Office
Office européen des brevets

(11) Veröffentlichungsnummer: **0 117 473**
**B1**

(12) # EUROPÄISCHE PATENTSCHRIFT

(45) Veröffentlichungstag der Patentschrift: **28.01.87**

(51) Int. Cl.⁴: **C 07 D 215/56**, C 07 D 401/12, A 61 K 31/495

(21) Anmeldenummer: **84101442.6**

(22) Anmeldetag: **13.02.84**

(54) **Chinoloncarbonsäuren, Verfahren zu ihrer Herstellung sowie diese enthaltende antibakterielle Mittel.**

(30) Priorität: **25.02.83 DE 3306771**

(43) Veröffentlichungstag der Anmeldung: **05.09.84 Patentblatt 84/36**

(45) Bekanntmachung des Hinweises auf die Patenterteilung: **28.01.87 Patentblatt 87/5**

(84) Benannte Vertragsstaaten: **AT BE CH DE FR GB IT LI LU NL SE**

(56) Entgegenhaltungen:
**EP - A - 0 004 279**
**EP - A - 0 049 355**
**EP - A - 0 078 362**
**EP - A - 0 090 424**
**DE - A - 2 840 910**

**CHEMICAL ABSTRACTS, Band 93, Nr. 23, 8. Dezember 1980, Columbus, Ohio, USA KYORIN PHARMACEUTICAL CO. "Substituted quinolinecarboxylic acid derivatives" Seite 535, Spalte 1, Auszug Nr. 220 772p**
**CHEMICAL ABSTRACTS, Band 93, Nr. 21, 24. November 1980, Columbus, Ohio, USA KOGA, HIROSHI "Structure-activity relationships of antibacterial 6,7-and 7,8-disubstituted**

(73) Patentinhaber: **BAYER AG, Konzernverwaltung RP Patentabteilung, D-5090 Leverkusen 1 Bayerwerk (DE)**

(72) Erfinder: **Petersen, Uwe, Dr., Auf dem Forst 4, D-5090 Leverkusen 1 (DE)**
Erfinder: **Grohe, Klaus, Dr., Am Wasserturm 10, D-5068 Odenthal (DE)**
Erfinder: **Kühle, Engelbert, Dr, von-Bodelschwingh-Strasse 42, D-5060 Bergisch-Gladbach 2 (DE)**
Erfinder: **Zeiler, Hans-Joachim, Dr., Elsbeekerstrasse 46, D-5620 Velbert 15 (DE)**
Erfinder: **Metzger, Karl Georg, Dr., Pahlkestrasse 75, D-5600 Wuppertal 1 (DE)**

(56) Entgegenhaltungen: (Fortsetzung)
**1-alkyl-1,4-dihydro-4-oxoquinoline-3-carboxylic acids" Seite 128, Spalte 1, Auszug Nr. 198 382n**

**Die Akte enthält technische Angaben, die nach dem Eingang der Anmeldung eingereicht wurden und die nicht in dieser Patentschrift enthalten sind.**

**EP 0 117 473 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Chinoloncarbonsäuren, Verfahren zu ihrer Herstellung sowie diese enthaltende antibakterielle Mittel.

In der EP-A1-0 049 355 und in der EP-A2-0 078 362 werden bereits Chinoloncarbonsäuren mit antibakterieller Wirkung beschrieben.

Es wurde gefunden, dass die neuen Chinoloncarbonsäuren der Formel (I)

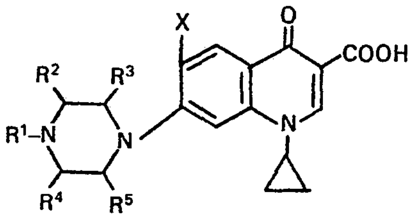

(I)

in der

$R^1$ für einen Rest $CO-R^6$, CN, $SO_2-R_7$ oder $S-R^8$ steht, wobei

$R^6$ Wasserstoff, gegebenenfalls durch 1 bis 3 Substituenten aus der Reihe Amino, Chlor, Alkoxycarbonyl mit 1 bis 4 Kohlenstoffatomen im Alkylteil, Carboxy, Alkoxy mit 1 bis 4 Kohlenstoffatomen, Hydroxy oder Trifluormethylthio substituiertes geradkettiges oder verzweigtes Alkyl mit 1 bis 5 C-Atomen, gegebenenfalls durch 1 bis 5 Substituenten aus der Reihe Fluor, Chlor, Hydroxy, Methoxy, Amino oder Carboxy substituiertes Phenyl, gegebenenfalls durch Amino substituiertes Benzyl, gegebenenfalls durch Fluor, Chlor, Pyrazol-1-yl, 1,2,3-Triazol-1-yl, N-Oxido-2-, -3- oder -4-pyridylmethyl substituiertes Alkoxy oder Alkylthio mit 1 bis 5 C-Atomen, Benzyloxy, Amino, gegebenenfalls durch Alkoxycarbonyl mit 1 bis 3 C-Atomen im Alkylteil, Benzyloxycarbonyl oder Carboxy substituiertes Alkylamino mit 1 bis 5 C-Atomen, Phenylamino,

$R^7$ gegebenenfalls durch 1 bis 3 Substituenten aus der Reihe Fluor oder Amino substituiertes geradkettiges oder verzweigtes Alkyl mit 1 bis 4 C-Atomen, Phenyl, Methylphenyl,

$R^8$ Methoxycarbonyl, Trichlormethyl, Trifluormethyl oder Dichlorfluormethyl bedeuten,

$R^2$, $R^3$, $R^4$ und $R^5$ gleich oder verschieden sein können und für Wasserstoff, Methyl, Ethyl, n- oder i-Propyl stehen, und

X für Wasserstoff, Halogen, vorzugsweise Fluor, oder Chlor, oder Nitro steht, und deren pharmazeutisch verwendbare Säureadditions-, Alkali- und Erdalkalisalze und Hydrate eine gute antibakterielle Wirkung sowohl gegen grampositive als auch gramnegative Bakterien aufweisen.

Bevorzugt sind solche Verbindungen der Formel (I), in denen die Symbole folgende Bedeutungen haben:

$R^1 = CO-R^6$, CN, $SO_2-R^7$, $S-R^8$;

$R^6$ = Wasserstoff, gegebenenfalls durch 1 bis 3 Substituenten aus der Reihe Amino, Chlor, Alkoxycarbonyl mit 1 bis 4 Kohlenstoffatomen im Alkylteil, Carboxy, Alkoxy mit 1 bis 4 Kohlenstoffatomen, Hydroxy- oder Trifluormethylthio substituiertes geradkettiges oder verzweigtes Alkyl mit 1 bis 5 C-Atomen, gegebenenfalls durch 1 bis 5 Substituenten aus der Reihe Fluor, Chlor, Hydroxy, Methoxy, Amino oder Carboxy substituiertes Phenyl, gegebenenfalls durch Amino substituiertes Benzyl, gegebenenfalls durch Fluor, Chlor, Pyrazol-1-yl, 1,2,3-Triazol-1-yl, N-Oxido-2-, -3- oder -4-pyridyl-methyl substituiertes Alkoxy oder Alkylthio mit 1 bis 5 C-Atomen, Benzyloxy, Amino, gegebenenfalls durch Alkoxycarbonyl mit 1 bis 3 C-Atomen im Alkylteil, Benzyloxycarbonyl oder Carboxy substituiertes Alkylamino mit 1 bis 5 C-Atomen, Phenylamino,

$R^7$ = gegebenenfalls durch 1 bis 3 Substituenten aus der Reihe Fluor oder Amino substituiertes geradkettiges oder verzweigtes Alkyl mit 1 bis 4 C-Atomen, Phenyl, Methylphenyl,

$R^8$ = Methoxycarbonyl, Trichlormethyl, Dichlorfluormethyl,

$R^2$, $R^3$, $R^4$ und $R^5$ = Wasserstoff, Methyl oder Ethyl und

X = Wasserstoff, Fluor, Chlor oder Nitro.

Besonders bevorzugt sind solche Verbindungen der Formel (I), in denen die Symbole folgende Bedeutungen haben:

$R^1 = CO-R^6$, CN, $SO_2R^7$, $S-R^8$,

$R^6$ = Wasserstoff, gegebenenfalls durch 1 oder 2 Substituenten aus der Reihe Amino, Alkoxycarbonyl mit 1 bis 3 Kohlenstoffatomen im Alkylteil, Carboxy, Alkoxy mit 1 bis 3 Kohlenstoffatomen oder Trifluormethylthio substituiertes geradkettiges oder verzweigtes Alkyl mit 1 bis 4 C-Atomen, gegebenenfalls durch 1 bis 5 Substituenten aus der Reihe Chlor, Hydroxy, Amino oder Carboxy substituiertes Phenyl, gegebenenfalls durch Amino substituiertes Benzyl, gegebenenfalls durch Pyrazol-1-yl, 1,2,3-Triazol-1-yl, N-Oxido-2-, -3- oder -4-pyridylmethyl substituiertes Alkoxy mit 1 bis 4 C-Atomen, Alkylthio mit 1 bis 2 C-Atomen, Benzyloxy, Amino, gegebenenfalls durch Alkoxycarbonyl mit 1 bis 3 C-Atomen im Alkylteil oder Carboxy substituiertes Alkylamino mit 1 bis 5 C-Atomen,

$R^7$ = geradkettiges oder verzweigtes Alkyl mit 1 bis 3 C-Atomen, Phenyl, Methylphenyl,

$R^8$ = Methoxycarbonyl, Trichlormethyl, Dichlorfluormethyl,

$R^2$ = Wasserstoff, Methyl, Ethyl,

$R^3$ = Wasserstoff,

$R^4$ = Wasserstoff, Methyl,

$R^5$ = Wasserstoff und

X = Wasserstoff, Fluor, Chlor oder Nitro.

Weiterhin wurde gefunden, dass man die erfindungsgemässen Verbindungen der Formel (I) erhält, wenn man eine Verbindung der Formel (II)

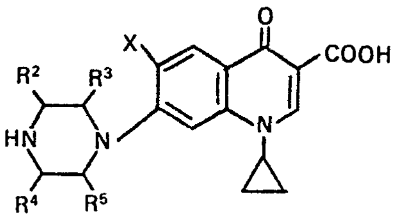

(II),

in welcher

X, $R^2$, $R^3$, $R^4$ und $R^5$ die oben angegebenen Bedeutungen haben, mit einer Verbindung der Formel (III)

$R^1$–Y (III),

in welcher

$R^1$ die oben angegebene Bedeutung hat und

Y für eine Abgangsgruppe wie Halogen, vorzugsweise Chlor, Fluor oder Brom, Methoxy, Ethoxy, Phenoxy, 4-Nitrophenoxy oder 2,3,5-Trichlorphenoxy, Alkoxycarbonyloxy steht, umsetzt (Methode A).

Des weiteren erhält man die erfindungsgemässen Verbindungen der Formel (I), wenn man Verbindungen der Formel (II)

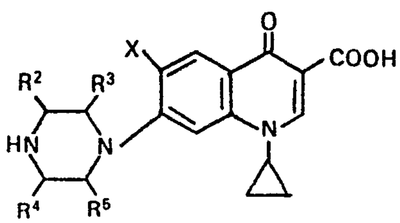

(II),

mit Isocyanaten der Formel (IV)

R'–NCO (IV)

in welcher

R' gegebenenfalls substituiertes Alkyl oder Phenyl bedeutet,

zu den erfindungsgemässen Verbindungen der Formel (Ia) = (I; $R^1$ = CO–NH–R') umsetzt (Methode B).

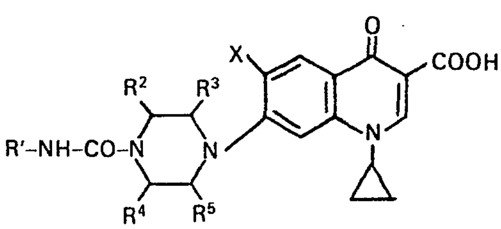

(Ia)

Man erhält die erfindungsgemässen Verbindungen der Formel (I) auch, wenn man Verbindungen der Formel (II)

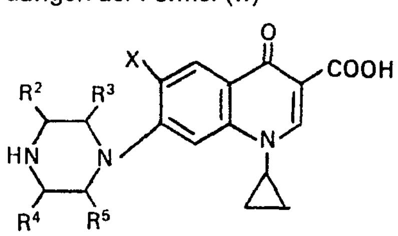

(II)

mit Anhydriden der Formel (V)

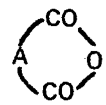

(V)

in welcher A eine gegebenenfalls substituierte Alkylenkette mit 2 oder 3 Kohlenstoffatomen oder einen Arylenrest bedeutet, zu den erfindungsgemässen Verbindungen der Formel (Ib) = (I; $R^1$ = CO–A–COOH) umsetzt (Methode C).

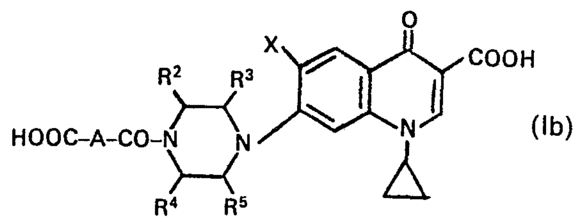

(Ib)

Überraschenderweise zeigen die erfindungsgemässen Chinoloncarbonsäuren eine erheblich höhere antibakterielle Wirkung als die bekannte 1-Ethyl-6-fluor-1,4- dihydro-4-oxo-7- (1-piperazinyl)- 3-chinolincarbonsäure (Norfloxacin). Die erfindungsgemässen Stoffe stellen somit eine Bereicherung der Pharmazie dar.

Verwendet man beispielsweise bei der Umsetzung von (II) mit (III) nach Methode A 1-Cyclopropyl-6-fluor- 1,4-dihydro-4-oxo-7- (1-piperazinyl)- 3-chinolincarbonsäure und Propionsäureanhydrid als Ausgangsverbindungen, so kann der Reaktionsablauf durch das folgende Formelschema wiedergegeben werden:

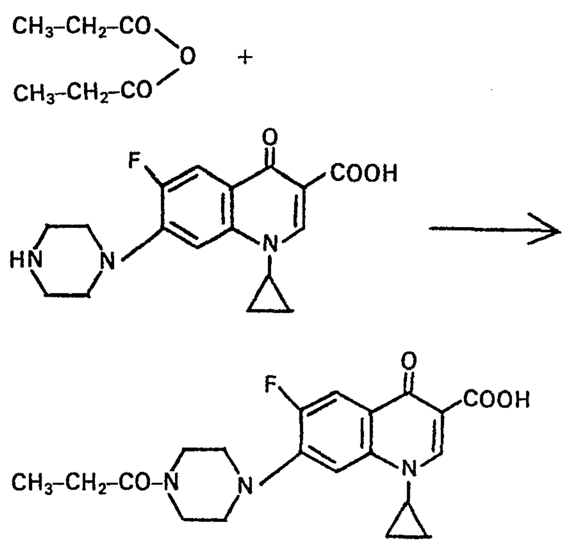

Verwendet man beispielsweise bei der Umsetzung von (II) mit (IV) nach Methode B 1-Cyclopropyl-6-fluor-1,4- dihydro-4-oxo-7- (1-piperazinyl)- 3-chinolincarbonsäure und Methylisocyanat als Ausgangssubstanzen, so kann der Reaktionsablauf durch das folgende Formelschema wiedergegeben werden:

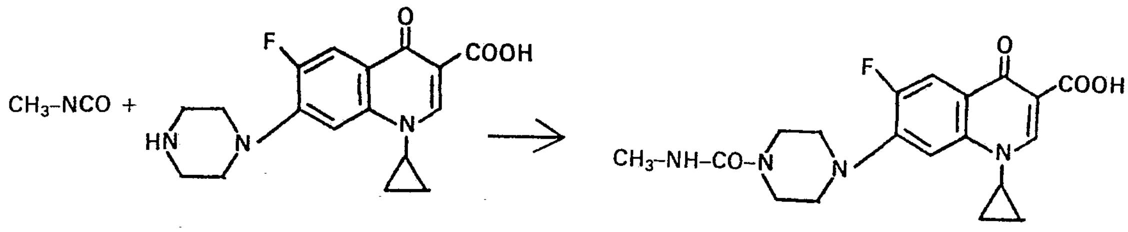

Verwendet man beispielsweise bei der Umsetzung von (II) mit (V) nach Methode C 1-Cyclopropyl-6-fluor-1,4- dihydro-4-oxo-7- (1-piperazinyl)-3- chinolincarbonsäure und Glutarsäureanhydrid als Ausgangsverbindungen, so kann der Reaktionsablauf durch das folgende Formelschema wiedergegeben werden:

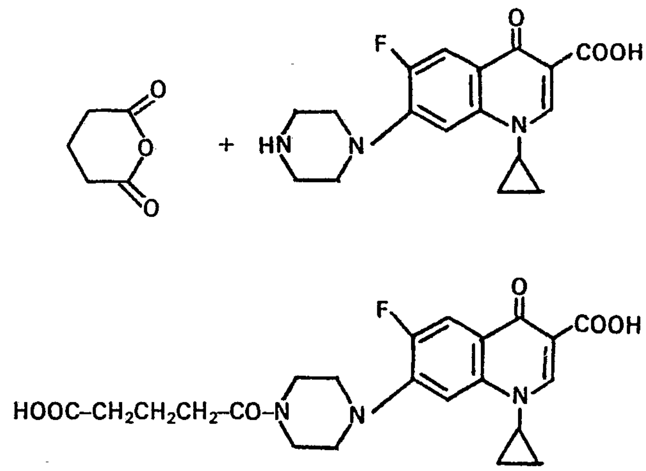

Die als Ausgangsverbindungen verwendeten Verbindungen der Formel (II) können durch Umsetzung von Verbindungen der Formel (VI)

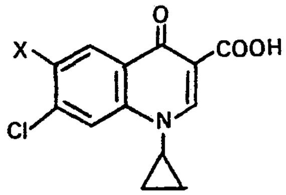

(VI)

mit Piperazin- oder Piperazinderivaten der Formel (VII)

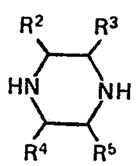

(VII)

hergestellt werden. Man arbeitet hierbei in einem Verdünnungsmittel, wie Dimethylsulfoxid, Hexamethylphosphorsäuretrisamid, Sulfolan, Wasser, einem Alkohol oder Pyridin bei Temperaturen von 20–200 °C, vorzugsweise bei 80–100 °C. Bei der Durchführung des Verfahrens setzt man auf 1 Mol Carbonsäure VI 1–15 Mol der Verbindung VII, vorzugsweise 1–6 Mol der Verbindung VII ein. Bei Verwendung äquivalenter Mengen der Carbonsäure VI und des Piperazinderivates VII führt man die Umsetzung in Gegenwart eines säurebindenden Mittels, beispielsweise Triethylamin, 1,4-Diaza-bicyclo[2.2.2]octan oder 1,8-Diaza-bicyclo[5.4.0]undec-7-en durch.

Als Beispiele für die auf diese Weise herstellbaren Ausgangsprodukte der Formel (II) seien genannt:

1-Cyclopropyl-6-fluor-1,4- dihydro-4-oxo-7-(1-piperazinyl)-3-chinolincarbonsäure,

1-Cyclopropyl-6-fluor-1,4-dihydro-4-oxo-7-(2,5-dimethyl-1-piperazinyl)- 3-chinolincarbonsäure,

1-Cyclopropyl-6-fluor-1,4-dihydro-4-oxo-7-(3,5-dimethyl-1- piperazinyl)- 3-chinolincarbonsäure,

1-Cyclopropyl-6-fluor-1,4-dihydro-4-oxo-7-(3-methyl-1-piperazinyl)- 3-chinolincarbonsäure,

1-Cyclopropyl-6-fluor-1,4-dihydro-4-oxo-7-(3-ethyl-1-piperazinyl)- 3-chinolincarbonsäure,

1-Cyclopropyl-6-fluor-1,4-dihydro-4-oxo-7-(3,5-diethyl-1-piperazinyl)- 3-chinolincarbonsäure,

1-Cyclopropyl-6-fluor-1,4-dihydro-4-oxo-7-(2,3,5-trimethyl-1-piperazinyl) -3-chinolincarbonsäure,

1-Cyclopropyl-6-fluor-1,4-dihydro-4-oxo-7-(2,3,5,6-tetramethyl-1-piperazinyl)- 3-chinolincarbonsäure,

1-Cyclopropyl-1,4-dihydro-4-oxo-7-(1-piperazinyl)-3- chinolincarbonsäure,

1-Cyclopropyl-1,4- dihydro-6-nitro-4-oxo- 7-(1-piperazinyl) -3-chinolincarbonsäure,

6-Chlor-1-cyclopropyl-1,4-dihydro-4-oxo-7-(1-piperazinyl)-3-chinolincarbonsäure.

Die als Zwischenprodukt verwendete 7-Chlor-1-cyclopropyl-6-fluor-1,4- dihydro-4-oxo-3-chinolincarbonsäure der Formel VIa (VI; X = F) kann gemäss folgendem Reaktionsschema hergestellt werden:

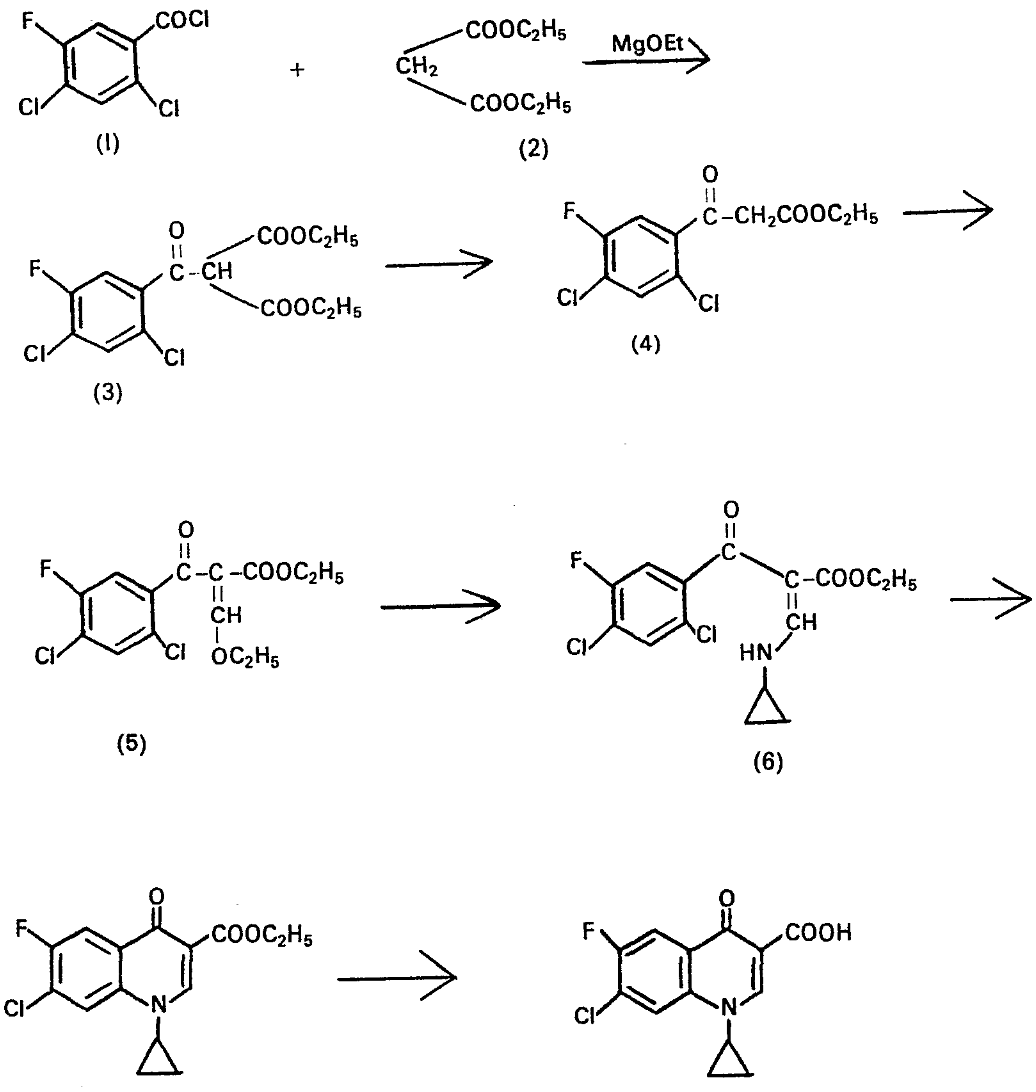


(1) (2) (3) (4) (5) (6) (7) (VIa)

Danach wird Malonsäurediethylester (2) mit (1) in Gegenwart von Magnesiumalkoholat mit 2,4-Dichlor-5-fluor- benzoylchlorid (1) [Deutsche Patentanmeldung Nr. 3 142 856.8] zum Acylmalonester (3) acyliert [Organikum, 3. Aufl. 1964, S. 438].

Durch partielle Verseifung und Decarboxylierung von (3) in wässrigem Medium mit katalytischen Mengen p-Toluolsulfonsäure erhält man in guter Ausbeute den Aroylessigsäureethylester (4), der mit o-Ameisensäuretriethylester/Acetanhydrid in den 2-(2,4-Dichlor-5-flluor-benzoyl)-3-ethoxy-acrylsäureethylester (5) übergeht. Die Umsetzung von (5) mit Cyclopropylamin in einem Lösungsmittel, wie z.B. Methylenchlorid, Alkohol, Chloroform, Cyclohexan oder Toluol, führt in leicht exothermer Reaktion zum gewünschten Zwischenprodukt (6).

Die Cyclisierungsreaktion (6) → (7) wird in einem Temperaturbereich von etwa 60 bis 280 °C, bevorzugt 80 bis 180 °C durchgeführt.

Als Verdünnungsmittel können Dioxan, Dimethylsulfoxid, N-Methyl-pyrrolidon, Sulfolan, Hexamethylphosphorsäuretriamid und bevorzugt N,N-Dimethylformamid verwendet werden.

Als Säurebinder kommen für diese Reaktionsstufe Kaliumtert.-butanolat, Butyl-lithium, Lithium-phenyl, Phenylmagnesiumbromid, Natriummethylat, Natriumhydrid und besonders bevorzugt Kalium- oder Natriumcarbonat in Betracht. Es kann vorteilhaft sein, einen Überschuss von 10 Mol-% an Base einzusetzen.

Die in dem letzten Schritt erfolgende Esterhydrolyse von (7) unter basischen oder sauren Bedingungen führt zur 7-Chlor-1-cyclopropyl-6-fluor -1,4-dihydro-4-oxo-3-chinolincarbonsäure VIa.

Das als Ausgangsmaterial für diesen Syntheseweg verwendete 2,4-Dichlor-5-fluor-benzoylchlorid (1) und die entsprechende Carbonsäure sowie das für die Herstellung von (1) benötigte 3-Fluor-4,6-dichlortoluol (10) werden gemäss nachstehendem Formelschema, ausgehend von 2,4-Dichlor-5-methyl-anilin (8), hergestellt:

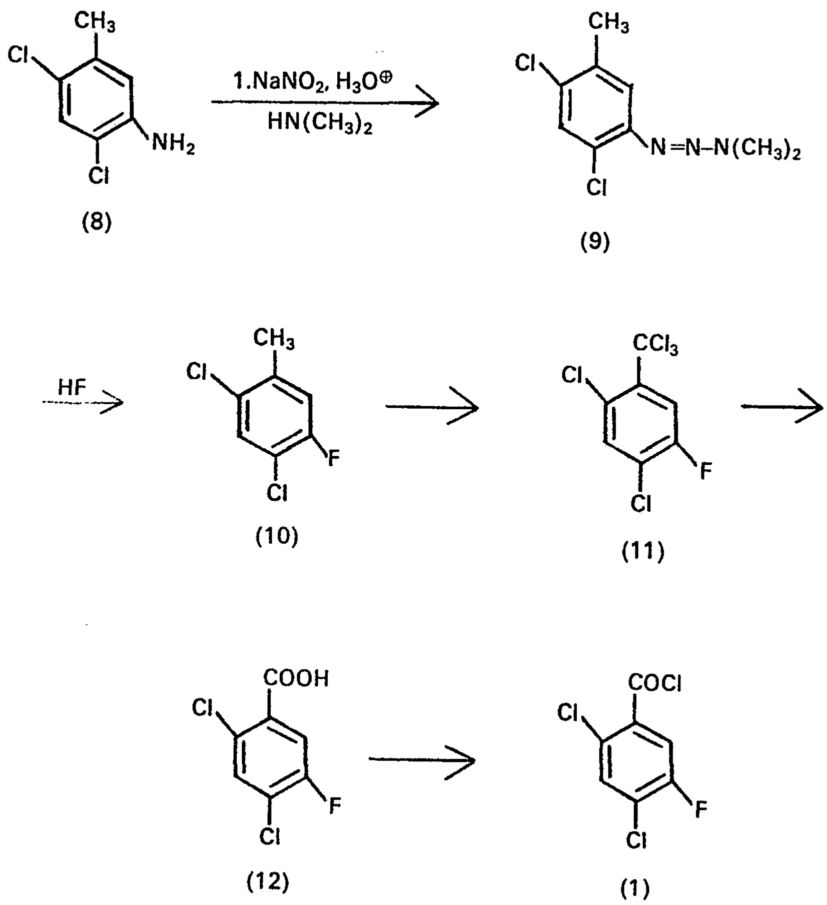

(8) (9) (10) (11) (12) (1)

Danach wird 2,4-Dichlor-5-methyl-anilin (8) mit Hilfe von $NaNO_2$ diazotiert und das dabei entstehende Diazoniumsalz mit Dimethylamin in das Triazen (9) übergeführt.

Das Triazin (9) wird in überschüssiger wasserfreier HF gelöst. Dabei spaltet das Triazen in 2,4-Dichlor-5-methyl- diazoniumfluorid und Dimethylamin. Ohne Zwischenisolierung wird diese Lösung thermisch bei 130–140 °C unter $N_2$-Abspaltung in 3-Fluor-4,6-dichlortoluol (10) gespalten. Ausbeute: 77,7% der Theorie.

Das 3-Fluor-4,6-dichlortoluol (10) wird in einem Temperaturbereich von 110–160 °C unter UV-Bestrahlung zu 2,4-Dichlor-5-fluor-1-trichlormethylbenzol (11) chloriert.

Die Verseifung von (11) mit 95%iger Schwefelsäure führt zu 2,4-Dichlor-5-fluor-benzoesäure (12), die mit Thionylchlorid in das Carbonsäurechlorid (1) (Sdp. 121°/20 mbar; $n_D^{20}$ 1,5722) übergeht.

Auf analoge Weise werden die folgenden als Zwischenprodukte verwendeten Chinoloncarbonsäuren hergestellt.

7-Chlor-1-cyclopropyl-1,4- dihydro-4-oxo-3-chinolincarbonsäure (VIb) (Schmp. 308 °C) aus 2,4-Dichlorbenzoylchlorid [J. Chem. Soc. 83, 1213 (1903)];

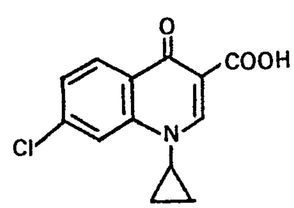
(VIb)

6,7-Dichlor-1- cyclopropyl-1,4- dihydro-4-oxo- 3-chinolincarbonsäure (VIc) (Schmp. 265 °C) aus 2,4,5-Trichlorbenzoylchlorid [Liebigs Ann. Chem. 152, 238 (1896)];

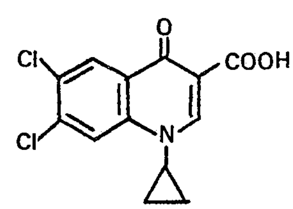
(VIc)

7-Chlor-1- cyclopropyl-1,4- dihydro-6-nitro-4- oxo-3-chinolincarbonsäure (VId) (Schmp. 265–275 °C Zers.) aus 2,4-Dichlor-5-nitro-benzoylchlorid [Liebigs Ann. Chem. 677, 8 (1964)].

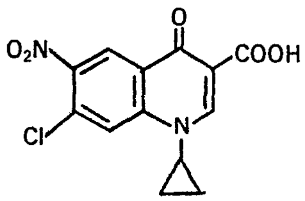

(VId)

Die erfindungsgemäss verwendbaren Verbindungen der Formel (III) sind bereits bekannt. Als Beispiele seien genannt:

Ameisensäureessigsäureanhydrid, Essigsäureanhydrid, Propionsäureanhydrid, Acetylchlorid, Chloracetylchlorid, Dichloracetylchlorid, Bromacetylbromid, Buttersäurechlorid, 4-Chlorbuttersäurechlorid, Isobuttersäurechlorid, 3-Methylbutansäurechlorid, Pentansäurechlorid, Benzoylchlorid, 3-Chlorbenzoylchlorid, 4-Fluorbenzoylchlorid, 4-Nitrobenzoylchlorid, 2-Chlor-4-nitrobenzoylchlorid, 4-Methylbenzoylchlorid, Bernsteinsäuremonomethylester-monochlorid, Trifluormethylthioessigsäurefluorid, N-(tert.-Butoxycarbonyl)-glycin- 4-nitrophenylester, N-(tert.-Butoxycarbonyl)-L-alanin- 4-nitrophenylester, N-(tert.-Butoxycarbonyl)-D-alanin- 2,4,5-trichlorphenylester, N-(tert.-Butoxycarbonyl)-L-leucin- 4-nitrophenylester, N-(tert.-Butoxycarbonyl)-L-valin- 4-nitrophenylester, 3-Methoxypropionsäurechlorid, Chlorkohlensäuremethylester, Chlorkohlensäureethylester, Chlorkohlensäure-n-butylester, Diethylcarbonat, Chlorcyan, Diphenylcarbonat, Bromcyan, Dimethyldicarbonat, Diethyldicarbonat, Di-tert.-butyl-dicarbonat, 4-Nitrophenyl-[2-(1-pyrazolyl)-ethyl]-carbonat, 4-Nitrophenyl-[2-(1-triazolyl)-ethyl]- carbonat, [(N-Oxido-2- pyridyl)-methyl]-4- nitrophenylcarbonat, [(N-Oxido-3-pyridyl)-methyl]-4-nitrophenyl-carbonat, [(N-Oxido-4-pyridyl)-methyl]-4-nitrophenyl-carbonat, Dimethylcarbamidsäurechlorid, Trichlormethansulfenylchlorid, Dichlorfluormethansulfenylchlorid, Trifluormethansulfenylchlorid, Methoxycarbonylsulfenylchlorid, Methansulfonsäurechlorid, Ethansulfonsäurechlorid, Propan-1-sulfonsäurechlorid, Benzolsulfonsäurechlorid, 4-Toluolsulfonsäurechlorid, Butan-1-sulfonsäurechlorid, Perfluorbutan-1- sulfonsäurefluorid, 4-Chlorbutan-1-sulfonsäurechlorid, Dichlorfluormethansulfonsäurechlorid.

Die erfindungsgemäss verwendbaren Isocyanate (IV) sind bekannt. Als Beispiel seien genannt:

Methylisocyanat, Ethylisocyanat, Isopropylisocyanat, 3-Isocyanatopropionsäure-n-propylester, 4-Isocyanatobuttersäuremethylester, 6-Isocyanatohexansäure-methylester, Phenylisocyanat, 4-Methoxyphenylisocyanat, 3-Chlorphenylisocyanat.

Die erfindungsgemäss verwendbaren Anhydride (V) sind bekannt. Als Beispiele seien genannt:

Bernsteinsäureanhydrid, Methylbernsteinsäureanhydrid, Glutarsäureanhydrid, Phthalsäureanhydrid, Tetrachlorphthalsäureanhydrid.

Die Umsetzung von (II) mit (III) (Methode A) wird vorzugsweise in einem Verdünnungsmittel, wie Dimethylsulfoxid, N,N-Dimethylformamid, Tetrahydrofuran, Sulfolan, Dioxan, Pyridin, Wasser oder auch in Gemischen dieser Verdünnungsmittel, bei Temperaturen von 0–140 °C, vorzugsweise 10–110 °C, vorgenommen.

Die Umsetzung kann bei Normaldruck, aber auch bei erhöhtem Druck, durchgeführt werden. Im allgemeinen arbeitet man bei Drucken zwischen etwa 1 und etwa 100 bar, vorzugsweise zwischen 1 und 10 bar.

Als Säurebinder können alle üblichen anorganischen und organischen Säurebindungsmittel verwendet werden. Hierzu gehören vorzugsweise die Alkalihydroxide, Alkalicarbonate, Pyridin und tert.-Amine wie Triethylamin, 1,4-Diazabicyclo-[2.2.2]octan.

Bei der Durchführung des erfindungsgemässen Verfahrens setzt man auf 1 Mol der Verbindung (II) 1 bis 4 Mol, vorzugsweise 1 bis 1,5 Mol der Verbindung (III) ein.

Die Umsetzung von (II) mit (IV) (Methode B) wird vorzugsweise in einem Verdünnungsmittel wie Dioxan, Dimethylsulfoxid, N,N-Dimethylformamid, verdünnter Natronlauge oder auch in Gemischen dieser Verdünnungsmittel durchgeführt.

Die Reaktionstemperaturen können in einem grösseren Bereich variiert werden. Im allgemeinen arbeitet man zwischen etwa 0 und etwa 100 °C, vorzugsweise zwischen 5 und 50 °C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemässen Verfahrens setzt man auf 1 Mol der Verbindung (II) 1–5 Mol, vorzugsweise 1–2 Mol, der Verbindung (IV) ein.

Die Umsetzung von (II) mit (V) (Methode C) wird in einem Verdünnungsmittel wie N,N-Dimethylformamid, Dioxan, Tetrahydrofuran, Pyridin, Wasser oder auch in Mischungen dieser Verdünnungsmittel durchgeführt. Die Reaktionstemperaturen können in einem grösseren Bereich variiert werden. Im allgemeinen arbeitet man zwischen etwa 0 und etwa 140 °C, vorzugsweise zwischen 10 und 100 °C.

Die Umsetzung kann bei Normaldruck, aber auch bei erhöhtem Druck, durchgeführt werden. Im allgemeinen arbeitet man bei Drucken zwischen etwa 1 und etwa 100 bar, vorzugsweise zwischen 1 und 10 bar.

Als Säurebinder können alla üblichen anorganischen und organischen Säurebindungsmittel verwendet werden. Hierzu gehören vorzugsweise die Alkalihydroxide, Alkalicarbonate, Pyridin und

tert.-Amine wie Triethylamin, 1,4-Diazabicyclo-[2.2.2]octan.

Bei der Durchführung des erfindungsgemässen Verfahrens setzt man auf 1 Mol der Verbindung (II) 1 bis 3 Mol, vorzugsweise 1 bis 1,3 Mol, der Verbindung (V) ein.

Als neue antibakterielle Wirkstoffe seien im einzelnen genannt:

7-[4-Formyl-1-piperazinyl]-1-cyclopropyl-6-fluor-1,4-dihydro-4-oxo-3-chinolincarbonsäure,

7-[4-Acetyl-1-piperazinyl]-1-cyclopropyl-6-fluor-1,4-dihydro-4-oxo-3-chinolincarbonsäure,

7-[4-Chloracetyl-1-piperazinyl]-1-cyclopropyl-6-fluor-1,4-dihydro-4-oxo-3-chinolincarbonsäure,

7-[4-(Trifluormethylthio-acetyl)-1-piperazinyl]-1-cyclopropyl-6-fluor-1,4-dihydro-4-oxo-3-chinolincarbonsäure,

7-[4-Propionyl-1-piperazinyl]-1-cyclopropyl-6-fluor-1,4-dihydro-4-oxo-3-chinolincarbonsäure,

7-[4-Butyryl-1-piperazinyl]-1-cyclopropyl-6-fluor-1,4-dihydro-4-oxo-chinolincarbonsäure,

7-[4-(4-Chlorbutyryl)-1-piperazinyl]-1-cyclopropyl-6-fluor-1,4-dihydro-4-oxo-3-chinolincarbonsäure,

7-[4-(3-Methyl-butyryl)-1-piperazinyl]-1-cyclopropyl-6-fluor-1,4-dihydro-4-oxo-3-chinolincarbonsäure

7-[4-(3-Methoxypropionyl)-1-piperazinyl]-1-cyclopropyl-6-fluor-1,4-dihydro-4-oxo-3-chinolincarbonsäure,

7-[4-Benzoyl-1-piperazinyl]-1-cyclopropyl-6-fluor-1,4-dihydro-4-oxo-3-chinolincarbonsäure,

7-[4-(4-Nitrobenzoyl)-1-piperazinyl]-1-cylopropyl-6-fluor-1,4-dihydro-4-oxo-3-chinolincarbonsäure,

7-[4-(4-Aminobenzoyl)-1-piperazinyl]-1-cyclopropyl-6-fluor-1,4-dihydro-4-oxo-3-chinolincarbonsäure,

7-[4-(3-Carboxypropionyl)-1-piperazinyl]-1-cyclopropyl-6-fluor-1,4-dihydro-4-oxo-3-chinolincarbonsäure,

7-[4-(4-Carboxybutyryl)-1-piperazinyl]-1-cyclopropyl-6-fluor-1,4-dihydro-4-oxo-3-chinilincarbonsäure,

7-[4-(2-Carboxybenzoyl)-1-piperazinyl]-1-cyclopropyl-6-fluor-1,4-dihydro-4-oxo-3-chinolincarbonsäure,

7-[4-(2-Carboxy-3,4,5,6-tetrachlorbenzoyl)-1-piperazinyl]-1-cyclopropyl-6-fluor-1,4-dihydro-4-oxo-3-chinolincarbonsäure,

7-[4-(3-Methoxycarbonyl-propionyl-1-piperazinyl]-1-cyclopropyl-6-fluor-1,4-dihydro-4-oxo-3-chinolincarbonsäure,

7-[4-(Hydroxyacetyl)-1-piperazinyl]-1-cyclopropyl-6-fluor-1,4-dihydro-4-oxo-3-chinolincarbonsäure,

7-[4-(Aminoacetyl)-1-piperazinyl]-1-cyclopropyl-6-fluor-1,4-dihydro-4-oxo-3-chinolincarbonsäure,

7-[4-(2-Aminopropionyl)-1-piperazinyl]-1-cyclopropyl-6-fluor-1,4-dihydro-4-oxo-3-chinolincarbonsäure,

7-[4-(2-Amino-3-methyl-butyryl)-1-piperazinyl]-1-cyclopropyl-6-fluor-1,4-dihydro-4-oxo-3-chinolincarbonsäure,

7-[4-(2-Amino-4-methyl-pentanoyl)-1-piperazinyl]-1-cyclopropyl-6-fluor-1,4-dihydro-4-oxo-3-chinolincarbonsäure,

7-[4-(2-Hydroxy-4-amino-butyryl)-1-piperazinyl]-1-cyclopropyl-6-fluor-1,4-dihydro-4-oxo-3-chinolincarbonsäure,

7-[4-Methoxycarbonyl-1-piperazinyl]-1-cyclopropyl-6-fluor-1,4-dihydro-4-oxo-3-chinolin carbonsäure,

7-[4-Ethoxycarbonyl-1-piperazinyl]-1-cyclopropyl-6-fluor-1,4-dihydro-4-oxo-3-chinolincarbonsäure,

7-[4-n-Butoxycarbonyl-1-piperazinyl]-1-cyclopropyl-6-fluor-1,4-dihydro-4-oxo-3-chinolincarbonsäure,

7-[4-tert.-Butoxycarbonyl-1-piperazinyl]-1-cyclopropyl-6-fluor-1,4-dihydro-4-oxo-3-chinolincarbonsäure,

7-{4-[2-(1-Pyrazolyl)-ethyloxycarbonyl]-1-piperazinyl}-1-cyclopropyl-6-fluor-1,4-dihydro-4-oxo-3-chinolincarbonsäure,

7-{4-[2-(1,2,3,-Triazol-1-yl)-ethoxycarbonyl]-1-piperazinyl}-1-cyclopropyl-6-fluor-1,4-dihydro-4-oxo-3-chinolincarbonsäure,

7-[4-(2-Chlorethyloxycarbonyl)-1-piperazinyl]-1-cyclopropyl-6-fluor-1,4-dihydro-4-oxo-3-chinolincarbonsäure,

7-{4-[(N-Oxido-3-pyridyl)-methyl]-1-piperazinyl}-1-cyclopropyl-6-fluor-1,4-dihydro-4-oxo-3-chinolincarbonsäure,

7-[4-Carbamoyl-1-piperazinyl]-1-cyclopropyl-6-fluor-1,4-dihydro-4-oxo-3-chinolincarbonsäure,

7-[4-Methylcarbamoyl-1-piperazinyl]-1-cyclopropyl-6-fluor-1,4-dihydro-4-oxo-3-chinolincarbonsäure,

7-[4-Hexylcarbamoyl-1-piperazinyl]-1-cyclopropyl-6-fluor-1,4-dihydro-4-oxo-3-chinolincarbonsäure,

7-[4-Phenylcarbamoyl]-1-cyclopropyl-6-fluor-1,4-dihydro-4-oxo-3-chinolincarbonsäure,

7-{4-[(3-Methoxycarbonylpropyl)-carbamoyl]-1-piperazinyl}-1-cyclopropyl-6-fluor-1,4-dihydro-4-oxo-3-chinolincarbonsäure,

7-{4-[(5-Methoxycarbonylpentyl)-carbamoyl]-1-piperazinyl}-1-cyclopropyl-6-fluor-1,4-dihydro-4-oxo-3-chinolincarbonsäure,

7-{4-[(3-Carboxypropyl)-carbamoyl]-1-piperazinyl}-1-cyclopropyl-6-fluor-1,4-dihydro-4-oxo-3-chinolincarbonsäure,

7-{4-[(5-Carboxypentyl)-carbamoyl]-1-piperazinyl}-1-cyclopropyl-6-fluor-1,4-dihydro-4-oxo-3-chinolincarbonsäure,

7-{4-[(2-Propoxycarbonyl)-carbamoyl]-1-piperazinyl}-1-cyclopropyl-6-fluor-1,4-dihydro-4-oxo-3-chinolincarbonsäure,

7-{4-[(2-Carboxyethyl)-carbamoyl]-1-piperazinyl}-1-cyclopropyl-6-fluor-1,4-dihydro-4-oxo-3-chinolincarbonsäure,

7-[4-Dimethylcarbamoyl-1-piperazinyl]-1-cyclopropyl- 6-fluor-1,4-dihydro-4-oxo-3-chinolincarbonsäure,

7-{4-[(4-Chlorbutyl)-carbamoyl] -1-piperazinyl}-1-cyclopropyl-6- fluor-1,4-dihydro-4-oxo-3-chinolincarbonsäure,

7-[4-Cyano-1-piperazinyl] -1-cyclopropyl-6-fluor-1,4- dihydro-4-oxo-3- chinolincarbonsäure,

7-[4-Trichlormethansulfenyl-1- piperazinyl] -1-cyclopropyl-6-fluor-1,4-dihydro- 4-oxo-3-chinolincarbonsäure,

7-[4-Methoxycarbonylsulfenyl-1-piperazinyl] -1-cyclopropyl-6-fluor-1,4- dihydro-4-oxo-3-chinolincarbonsäure,

7-[4-Methansulfonyl-1-piperazinyl] -1-cyclopropyl-6-fluor-1,4-dihydro-4- oxo-3-chinolincarbonsäure,

7-[4-Dichlorfluormethansulfonyl-1-piperazinyl] -1-cyclopropyl-6-fluor- 1,4-dihydro-4-oxo-3- chinolincarbonsäure,

7-[4-n-Propansulfonyl-1-piperazinyl] -1-cyclopropyl-6-fluor-1,4-dihydro-4- oxo-3-chinolincarbonsäure,

7-[4-(4-Chlorbutan-1-sulfonyl-) -1-piperazinyl] -1-cyclopropyl-6-fluor-1,4- dihydro-4-oxo-3- chinolincarbonsäure,

7-[4-Perfluorbutan-1-sulfonyl-1- piperazinyl] -1-cyclopropyl-6-fluor-1,4- dihydro-4-oxo- 3-chinolincarbonsäure,

7-[4-Acetyl-3-methyl-1-piperazinyl] -1-cyclopropyl-6-fluor -1,4-dihydro-4-oxo- 3-chinolincarbonsäure,

7-[3-Methyl-4-propionyl-1-piperazinyl] -1-cyclopropyl-6- fluor-1,4-dihydro-4-oxo- 3-chinolincarbonsäure,

7-[3-Methyl-4-methylcarbamoyl-1-piperazinyl] -1-cyclopropyl-6- fluor-1,4-dihydro-4-oxo-3-chinolincarbonsäure,

7-[2,5-Dimethyl-4-formyl-1-piperazinyl]-1-cyclopropyl-6-fluor- 1,4-dihydro-4-oxo- 3-chinolincarbonsäure,

7-[2,5-Dimethyl-4-butyryl-1-Piperazinyl]-1-cyclopropyl-6-Pluor-1,4-dihydro-4-oxo-3-chinolincarbonsäure,

7-[3,5-Dimethyl-4-acetyl-1-piperazinyl] -1-cyclopropyl-6-fluor-1,4-dihydro-4-oxo- 3-chinolincarbonsäure,

7-[3,5-Dimethyl-4-carbamoyl-1- piperazinyl] -1-cyclopropyl-6-fluor- 1,4-dihydro-4-oxo-3-chinolincarbonsäure,

7-[4-Butyryl-1-piperazinyl) -1-cyclopropyl-1,4- dihydro-6-nitro-4- oxo-3-chinolincarbonsäure,

7-(4-Butyryl-1-piperazinyl) -6-chlor-1-cyclopropyl- 1,4-dihydro-4-oxo-3-chinolincarbonsäure,

7-(4-Butyryl-1-piperazinyl)- 1-cyclopropyl-1,4- dihydro-4- oxo-3-chinolincarbonsäure,

7-[4-(2-Aminopropionyl)- 3-methyl-1- piperazinyl]- 1-cyclopropyl-6- fluor-1,4-dihydro-4-oxo- 3-chinolincarbonsäure,

7-(4-Butyryl-3,5-dimethyl-1-piperazinyl)-1-cyclopropyl-6- fluor-1,4-dihydro-4-oxo-3-chinolincarbonsäure.

Die erfindungsgemässen Verbindungen der Formel (I) können gegebenenfalls mit einer organischen oder anorganischen Säure in ein Salz übergeführt werden. Zur Salzbildung geeignete Säuren sind z.B. Halogenwasserstoffsäuren wie Salzsäure, Bromwasserstoffsäure, Jodwasserstoffsäure, Schwefelsäure, Essigsäure, Citronensäure, Ascorbinsäure, Methansulfonsäure und Benzolsulfonsäure. Als Alkali bzw. Erdalkalisalze sind vorzugsweise Natrium-, Kalium-, Calcium- und Magnesiumsalze geeignet.

Herstellungsbeispiele für die Ausgangsverbindungen:

Beispiel A

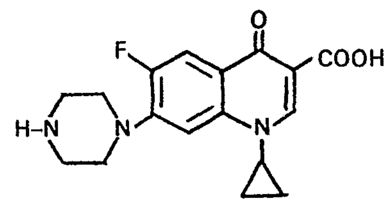

Ein Gemisch von 19,7 g 7-Chlor-1-cyclopropyl- 6-fluor-1,4-dihydro-4-oxo- 3-chinolincarbonsäure, 30,1 g wasserfreiem Piperazin und 100 ml Dimethylsulfoxid wird 2 Stunden auf 135–140 °C erhitzt. Das Lösungsmittel wird im Feinvakuum abdestilliert, der Rückstand in $H_2O$ suspendiert, abgesaugt und mit Wasser gewaschen. Zur weiteren Reinigung wird das feuchte Rohprodukt mit 100 ml Wasser aufgekocht, bei Raumtemperatur abgesaugt, mit $H_2O$ gewaschen und im Vakuumtrockenschrank über $CaCl_2$ bei 100 °C bis zur Gewichtskonstanz getrocknet. Man erhält 19,6 g 1-Cyclopropyl-6-fluor-1,4- dihydro-4-oxo- 7-(1-piperazinyl) -3-chinolincarbonsäure vom Zersetzungspunkt 255–257 °C.

Die als Ausgangsmaterial verwendete 7-Chlor-1-cyclopropyl-6-fluor-1,4- dihydro-4-oxo-3-chinolincarbonsäure VIa wird wie folgt hergestellt:

24,3 g Magnesiumspäne werden in 50 ml wasserfreiem Ethanol suspendiert. Man versetzt mit 5 ml Tetrachlorkohlenstoff und tropft, wenn die Reaktion in Gang gekommen ist, ein Gemisch von 160 g Malonsäurediethylester, 100 ml absolutem Ethanol und 400 ml wasserfreiem Ether zu, wobei ein heftiger Rückfluss zu beobachten ist. Nach dem Abklingen der Reaktion wird noch 2 Stunden zum Sieden erhitzt, mit Trockeneis/Aceton auf –5 bis –10 °C gekühlt und bei dieser Temperatur eine Lösung von 227,5 g 2,4-Dichlor-5-fluor- benzoylchlorid (1) in 100 ml abs. Ether langsam zugetropft. Man rührt 1 Stunde bei 0 bis –5 °C, lässt über Nacht auf Raumtemperatur kommen und lässt unter Eiskühlung ein Gemisch von 400 ml Eiswasser und 25 ml konz. Schwefelsäure zulaufen. Die Phasen werden getrennt und zweimal mit Ether nachextrahiert. Die vereinigten

Etherlösungen werden mit gesättigter NaCl-Lösung gewaschen, mit $Na_2SO_4$ getrocknet und das Lösungsmittel i.Vak. abgezogen. Man erhält 349,5 g 2,4-Dichlor-5-fluor- benzoylmalonsäure-diethylester (3) als Rohprodukt.

Eine Emulsion von 34,9 g rohem 2,4-Dichlor-5-fluor- benzoyl-malonsäure-diethylester (3) in 50 ml Wasser wird mit 0,15 g p-Toluolsulfonsäure versetzt. Man erhitzt unter gutem Rühren 3 Stunden zum Sieden, extrahiert die erkaltete Emulsion mehrmals mit Methylenchlorid, wäscht die vereinigten $CH_2Cl_2$-Lösungen einmal mit gesättigter NaCl-Lösung, trocknet mit $Na_2SO_4$ und destilliert das Lösungsmittel i.Vak. ab. Die Fraktionierung des Rückstands i.Vak. liefert 21,8 g 2,4-Dichlor-5-fluor-benzoyl- essigsäureethylester (4) vom Sdp. 127–142 °C/0,09 mbar.

Ein Gemisch von 21,1 g 2,4-Dichlor-5-fluorbenzoyl-essigsäureethylester (4), 16,65 g o-Ameisensäureethylester und 18,55 g Acetanhydrid wird 2 Stunden auf 150 °C erhitzt. Dann werden im Wasserstrahlvakuum und zuletzt im Feinvakuum bei einer Badtemperatur von 120 °C die flüchtigen Bestandteile abdestilliert. Zurück bleiben 25,2 g roher 2-(2,4-Dichlor-5-benzoyl)-3-ethoxy-acrylsäureethylester (5). Es ist genügend rein für die weiteren Umsetzungen.

Eine Lösung von 24,9 g 2-(2,4-Dichlor-5-fluor-benzoyl)- 3-ethoxy-acrylsäureethylester (5) in 80 ml Ethanol wird unter Eiskühlung und Rühren tropfenweise mit 4,3 g Cyclopropylamin versetzt. Wenn die exotherme Reaktion abgeklungen ist, wird noch 1 Stunde bei Raumtemperatur gerührt, das Lösungsmittel i. Vak. abgezogen und der Rückstand aus Cyclohexan/Petrolether umkristallisiert. Man erhält 22,9 g 2-(2,4-Dichlor-5-fluor-benzoyl)-3-cyclopropylamino-acrylsäureethylester (6) vom Schmp. 89–90 °C.

Eine Lösung von 31,9 g 2-(2,4-Dichlor-5-fluor-benzoyl)-3- cyclopropylamino- acrylsäureethylester (6) in 100 ml wasserfreiem Dioxan wird unter Eiskühlung und Rühren portionsweise mit 3,44 g 80-proz. Natriumhydrid versetzt. Dann wird 30 Min. bei Raumtemperatur und 2 Stunden unter Rückfluss gerührt und das Dioxan im Vakuum abgezogen. Der Rückstand (40,3 g) wird in 150 ml Wasser suspendiert, mit 6,65 g Ätzalkali versetzt und 1,5 Stunden refluxiert. Man filtriert die warme Lösung und wäscht mit $H_2O$ nach. Dann wird mit halbkonz. Salzsäure unter Eiskühlung auf pH = 1–2 angesäuert, der Niederschlag abgesaugt, mit Wasser gewaschen und i.Vak. bei 100 °C getrocknet. Man erhält auf diese Weise 27,7 g 7-Chlor-1-cyclopropyl-6-fluor-1,4-dihydro- 4-oxo-3-chinolincarbonsäure VIa vom Schmp. 234–237 °C.

Beispiel B

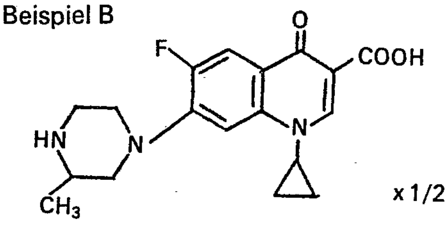

x 1/2 $H_2O$

Ein Gemisch aus 2,8 g (0,01 Mol) 7-Chlor-1-cyclopropyl-6-fluor- 1,4-dihydro-4- oxo-3-chinolincarbonsäure und 5,1 g (0,051 Mol) 2-Methylpiperazin in 6 ml Dimethylsulfoxid wird 2 Stunden auf 140 °C erhitzt. Anschliessend wird das Lösungsmittel im Hochvakuum abdestilliert, der Rückstand mit 6 ml heissem Wasser versetzt und 1 Stunde auf 95 °C gehalten. Man kühlt mit Eis, saugt den ausgefallenen Niederschlag ab, wäscht mit etwas Wasser und löst ihn in einer Mischung von 0,8 ml Essigsäure und 10 ml Wasser bei 90–100 °C auf. Das Filtrat wird mit Kalilauge (0,75 g KOH in 0,7 ml Wasser) auf pH 8 gebracht und der ausgefallene Niederschlag aus Methanol umkristallisiert. Man erhält 1,8 g (52% der Theorie) 1-Cyclopropyl-6-fluor-1,4-dihydro- 4-oxo-7-(3-methyl-1-piperazinyl)- 3-chinolincarbonsäure-semihydrat mit einem Zersetzungspunkt von 230–232 °C.

Beispiel C

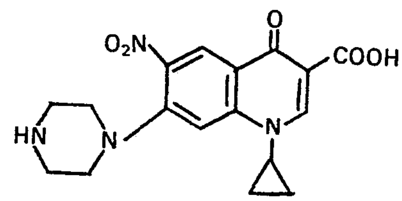

Eine Mischung von 9,3 g (0,03 Mol) 7-Chlor--1-clopropyl-1,4-dihydro-6-nitro-4-oxo-3-chinolincarbonsäure und 12,9 g (0,15 Mol) Piperazin wird in 60 ml Dimethylsulfoxid 15 Minuten auf 120 °C erwärmt. Aus der heissen Lösung fällt nach kurzer Zeit ein Niederschlag aus. Man engt im Hochvakuum ein, verrührt mit 30 ml Wasser und erhitzt noch 30 Minuten auf 95 °C. Mit 2n Salzsäure wird die Mischung auf pH 8 eingestellt, der Niederschlagabgesaugt und mit Wasser und Methanol gewaschen. Man isoliert 5,8 g (54% d.Th.)
1-Cyclopropyl-1,4-dihydro-6-nitro-4-oxo-7-(1-piperazinyl)-3-chinolincarbonsäure mit einem Zersetzungspunkt von 296–298 °C.

Beispiel D

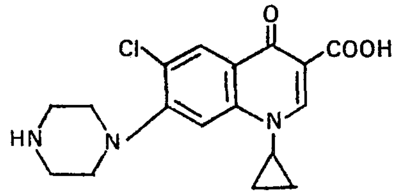

Man setzt analog Beispiel C 6,7-Dichlor-1-cyclopropyl-1,4-dihydro- 4-oxo-3-chinolincarbonsäure zu 1-Cyclopropyl-6-chlor-1,4-dihydro-4-oxo-7-(1-piperazinyl)- 3-chinolincarbonsäure mit einem Zersetzungspunkt von 295–298 °C um.

Beispiel E

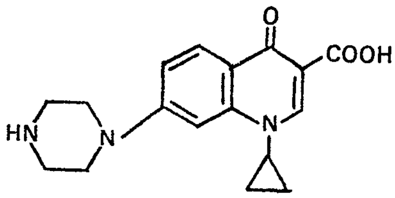

Man setzt analog Beispiel C 7-Chlor-1-cyclopropyl-1,4-dihydro-4-oxo-3-chinolincarbonsäure mit Piperazin zu 1-Cyclopropyl-1,4-dihydro-4-oxo-7- (1-piperazinyl)-3-chinolincarbonsäure mit einem Zersetzungspunkt von 298–300 °C um.

Herstellungsbeispiele für die erfindungsgemässen Endprodukte:

Beispiel 1

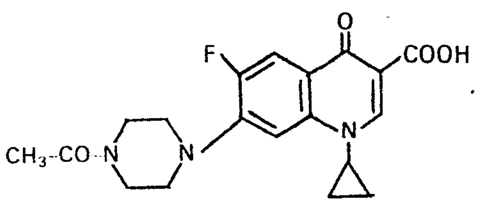

3,3 g (0,01 Mol) 1-Cyclopropyl-6-fluor-1,4-dihydro-4-oxo-7- (1-piperazinyl) -3-chinolincarbonsäure wird in einer Mischung aus 20 ml Dioxan und einer Lösung von 0,4 g Natriumhydroxid in 5 ml Wasser gelöst. Hierzu tropft man unter Eiskühlung gleichzeitig eine Lösung von 0,9 g (0,011 Mol) Acetylchlorid in 5 ml Dioxan und eine Lösung von 0,4 g (0,01 Mol) Natriumhydroxid in 5 ml Wasser zu, wobei der pH-Wert >8 gehalten wird. Man lässt 2 Stunden bei Raumtemperatur nachrühren, versetzt die Suspension mit 30 ml Wasser, säuert mit 2n Salzsäure an, saugt den Niederschlag ab und kristallisiert aus Glykolmonomethylether um. Es werden 2 g (54% der Theorie) 7-(4-Acetyl-1-piperazinyl) -1-cyclopropyl-6-fluor- 1,4-dihydro-4-oxo-3-chinolincarbonsäure mit einem Zersetzungspunkt von 267–270 °C isoliert.

Entsprechend Beispiel 1 werden die folgenden Verbindungen erhalten:

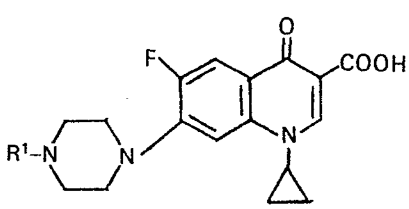

| Beispiel | $R^1$ | Schmelzpunkt °C |
|---|---|---|
| 2 | $CH_3CH_2$–CO | 267 (Zers.) |
| 3 | $CH_3CH_2CH_2$–CO | 282 (Zers.) |
| 4 | $\genfrac{}{}{0pt}{}{CH_3}{CH_3}{>}CH{-}CH_2{-}CO$ | 286 (Zers.) |
| 5 | $CF_3$–S–$CH_2$–CO | 252 (Zers.) |
| 6 | 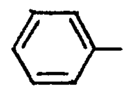CO | 324 (Zers.) |
| 7 | $CH_3O$–CO–$CH_2CH_2$–CO | 205 |
| 8 | CN | 276 (Zers.) |
| 9 | $CH_3O$–CO | 270 (Zers.) |
| 10 | $C_2H_5OCO$ | 315 (Zers.) |
| 11 | n–$C_4H_9OCO$ | 245 |
| 12 | 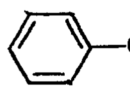$CH_2O$–CO | 230 |
| 12a | $C_2H_5$–S–CO | 322 (Zers.) |
| 13 | $CH_3$–$SO_2$ | 305 (Zers.) |
| 14 | n–$C_3H_7$–$SO_2$ | 268 (Zers.) |
| 15 | $CFCl_2$–$SO_2$ | 278 (Zers.) |
| 16 | $CCl_3$–S | 172 (Zers.) |
| 17 | $CFCl_2$–S | 188 (Zers.) |
| 18 | $CH_3O$–CO–S | 204 (Zers.) |
| 19 | $CH_3CH_2CH_2$–O–$CH_2$–CO | 220 |

Beispiel 20

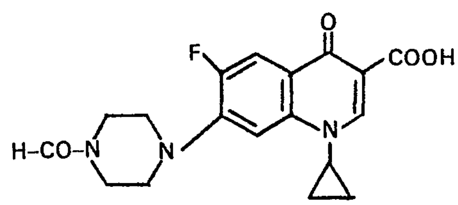

Man arbeitet wie in Beispiel 1 unter Verwendung von Ameisensäure-essigsäure-anhydrid und erhält 1-Cyclopropyl-6-fluor-7- (4-formyl-1-piperazinyl) -1,4-dihydro-4-oxo- 3-chinolincarbonsäure mit einem Zersetzungspunkt von 278–281 °C.

Beispiel 21

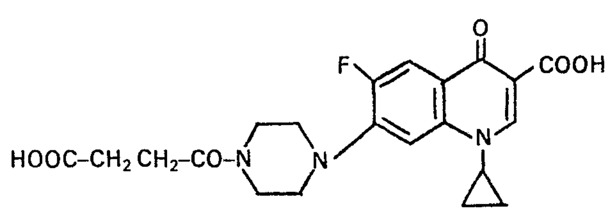

3,3 g (0,01 Mol) 1-Cyclopropyl-6-fluor-1,4-dihydro-4-oxo-7- (1-piperazinyl) -3-chinolincarbonsäure werden in einer Lösung von 0,4 g Natriumhydroxid in 20 ml Wasser gelöst und bei Raumtemperatur gleichzeitig mit einer Lösung von 1 g Bernsteinsäureanhydrid in 10 ml Dioxan und 0,4 g Natriumhydroxid in 10 ml Wasser versetzt. Man lässt 2 Stunden bei Raumtemperatur nachrühren, säuert mit 2n Salzsäure an, saugt den ausgefallenen Niederschlag ab und wäscht ihn mit Wasser und Methanol. Es werden 3,4 g (79% der Theorie) 7-[4-(3-Carboxypropionyl) -1-piperazinyl] -1-cyclopropyl-6-fluor- 1,4-dihydro-4-oxo- 3-chinolincarbonsäure mit einem Zersetzungspunkt von 284–286 °C erhalten.

Entsprechend Beispiel 21 werden die folgenden Verbindungen erhalten:

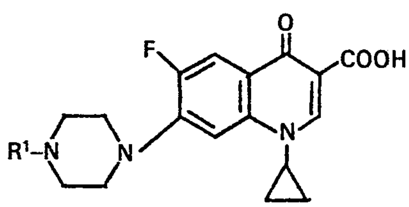

| Beispiel | $R^1$ | Schmelzpunkt °C |
|---|---|---|
| 22 | $HOOC-CH_2CH_2CH_2-CO-$ | 273 |
| 23 | 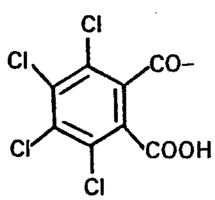 | 253 (Zers.) |

Beispiel 24

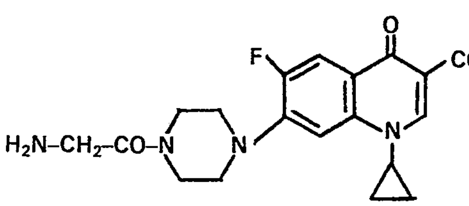

x 1/2 $H_2O$

3,3 g (0,01 Mol) 1-Cyclopropyl-6-fluor-1,4-dihydro-4-oxo-7- (1-piperazinyl)-3-chinolincarbonsäure werden in 50 ml Pyridin mit 3 g N-(tert.- Butoxycarbonyl)- glycin-4- nitro-phenylester versetzt und 4 Stunden bei Raumtemperatur gerührt. Die Lösung wird eingeengt, mit 30 ml Wasser versetzt und mit 2n Salzsäure auf pH 5 eingestellt. Der ausgefallene Niederschlag wird abgesaugt, mit Wasser und Methanol gewaschen und getrocknet. Man erhält 2,7 g (55% der Theorie) 7-[4-(tert.-Butoxycarbonylamino-acetyl)-1-piperazinyl]- 1-cyclopropyl-6-fluor-1,4-dihydro-4-oxo-3-chinolincarbonsäure mit einem Zersetzungspunkt von 236–238 °C. Eine Suspension von 2,2 g (0,0045 Mol) dieses Zwischenproduktes in 100 ml Methanol wird mit 10 ml konzentrierter Salzsäure versetzt und 3 Stunden bei Raumtemperatur stehengelassen. Danach werden Methanol und überschüssiger Chlorwasserstoff im Vakuum abgezogen, die wässrige Lösung mit verdünnter Natronlauge auf pH 8 eingestellt, der Niederschlag abgesaugt und mit Methanol gewaschen. Es werden 1,4 g (78% der Theorie) 7-(Aminoacetyl)-1-cyclopropyl-6-fluor-1,4-dihydro-4-oxo- 3-chinolincarbonsäure-semihydrat mit einem Zersetzungspunkt von 245–248 °C erhalten.

Analog Beispiel 24 werden folgende Verbindungen hergestellt:

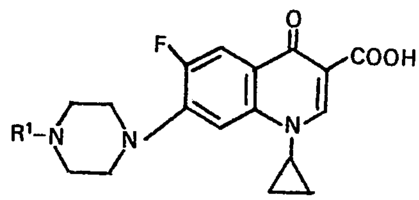

| Beispiel | $R^1$ | Zersetzungspunkt °C |
|---|---|---|
| 25 | 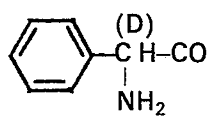 xHCl x $H_2O$ | 297 |
| 26 | 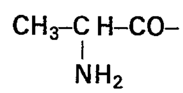 | 240 |
| 27 | 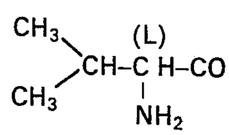 x HCl | 280 |
| 28 | $H_2N-CH_2-CH_2-CO-$ x HCl | 274 |

Beispiel 29

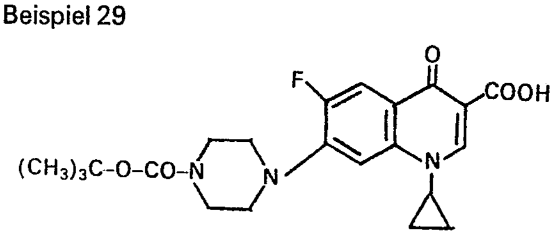

Eine Lösung von 8,25 g (0,025 Mol) 1-Cyclopropyl-6-fluor-1,4-dihydro-4-oxo-7- (1-piperazinyl)-3-chinolincarbonsäure in 75 ml Dioxan/Wasser (2:1) und 25 ml 1n-Natronlauge wird unter Eiskühlung mit 6 g Pyrokohlensäure-di-tert.-butylester versetzt und die Mischung

dann 30 Minuten bei Raumtemperatur gerührt. Man engt bis auf ein Drittel des Volumens ein, überschichtet mit 50 ml Essigsäureethylester und säuert mit verdünnter Kaliumhydrogensulfatlösung bis pH 3 an. Der ausgefallene Niederschlag wird abgesaugt, mit Wasser und Methanol gewaschen und im Hochvakuum getrocknet. Man erhält 10 g (92% der Theorie) 7-(4-tert.-Butoxycarbonyl-1-piperazinyl)- 1-cyclopropyl-6-fluor-1,4-dihydro-4- oxo- 3-chinolincarbonsäure mit einem Zersetzungspunkt von 249–252 °C.

Beispiel 30

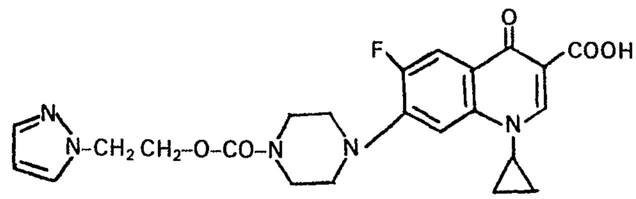

Eine Mischung von 3,3 g (0,01 Mol) 1-Cyclopropyl-6-fluor- 1,4-dihydro-4-oxo-7- (1-piperazinyl)-3-chinolincarbonsäure und 2,8 g (0,01 Mol) 4-Nitrophenyl-[2-(1-pyrazolyl)-ethyl]-carbonat in 40 ml Pyridin wird 1 Tag bei Raumtemperatur gerührt. Danach wird mit 30 ml Wasser verdünnt, mit 2n Salzsäure angesäuert, der Niederschlag abgesaugt und aus Glykolmonomethylether umkristallisiert. Man erhält 2,8 g (60% der Theorie) 1-Cyclopropyl-6-fluor- 1,4-dihydro-4-oxo-7-{-4-[2-(1-pyrazolyl)-ethyloxycarbonyl]-1-piperazinyl}- 3-chinolincarbonsäure mit einem Schmelzpunkt von 205–208 °C.

Das als Ausgangsprodukt eingesetzte 4-Nitrophenyl-[2-(1-pyrazolyl)-ethyl]-carbonat wird auf folgendem Wege erhalten: 4,6 g 1-(2-Hydroxyethyl)-pyrazol werden in 80 ml Acetonitril mit 4 g Chlorkohlensäure-4-nitrophenylester bei Raumtemperatur 12 Stunden gerührt, die Lösung eingeengt das erhaltene Öl in Methylenchlorid aufgenommen und mit Wasser gewaschen. Man trocknet mit Natriumsulfat, engt ein und erhält das rohe Carbonat als zähes Öl.

Analog Beispiel 30 werden die folgenden Verbindungen erhalten:

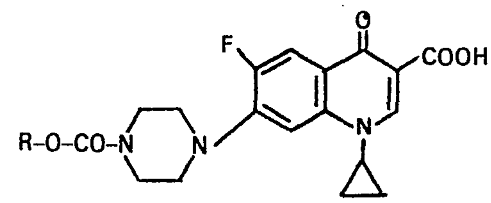

| Beispiel | R | Schmelzpunkt °C |
|---|---|---|
| 31 | 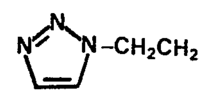 | 226 |
| 32 | 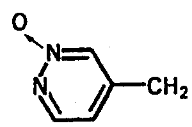 | 236 (Zers.) |
| 33 | 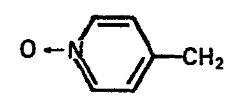 | 203 (Zers.) |

Beispiel 34

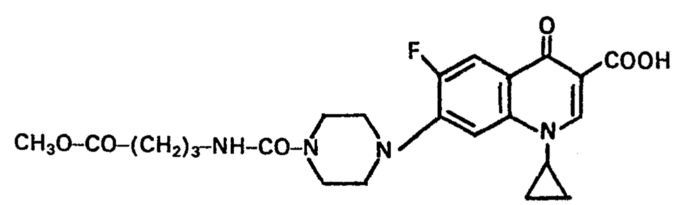

6,6 g (0,02 Mol) 1-Cyclopropyl-6-fluor-1,4-dihydro-4-oxo-7- (1-piperazinyl) -3-chinolincarbonsäure werden in einer Lösung von 0,4 g Natriumhydroxid in 40 ml Wasser gelöst und unter Eiskühlung mit einer Lösung von 3,2 g (0,022 Mol) 4-Isocyanato-buttersäuremethylester in 12 ml Dioxan versetzt. Danach wird 2 Stunden bei Raumtemperatur nachgerührt, mit 2n Salzsäure auf pH 5 eingestellt, der Niederschlag abgesaugt und aus Methanol umkristallisiert. Man erhält 6 g (63% der Theorie) 1-Cyclopropyl-6-fluor-1,4- dihydro-4-oxo-7- {4-[(3-methoxycarbonylpropyl) -carbamoyl] -1-piperazinyl}-3-chinolincarbonsäure mit einem Schmelzpunkt von 198–200 °C.

Entsprechend Beispiel 34 werden die folgenden Verbindungen erhalten:

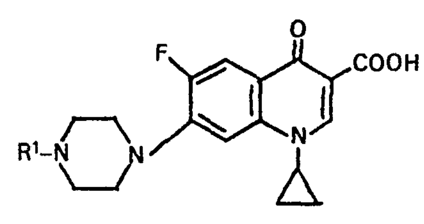

$R^1$

| Beispiel | $R^1$ | Schmelzpunkt (°C) |
|---|---|---|
| 35 | $CH_3O-CO-(CH_2)_5-NH-CO$ | 178 |
| 36 | $C_3H_7O-CO-(CH_2)_2-NH-CO$ | 180 (Zers.) |
| 37 | $CH_3-NH-CO$ | 280 (Zers.) |

Beispiel 38

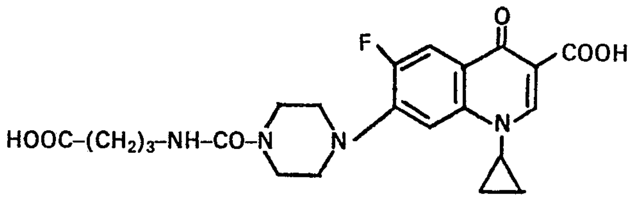

2,37 g der Verbindung des Beispiel 34 werden in einer Mischung aus 10 ml Eisessig, 6,5 ml Wasser und 1 ml konzentrierter Schwefelsäure, 1,5 Stunden auf 150–160 °C erhitzt. Nach dem Abkühlen auf Raumtemperatur wird auf 50 ml Eiswasser ausgegossen, der Niederschlag abgesaugt und aus Glykolmonomethylether umkristallisiert. Man isoliert 1,2 g (52% der Theorie) 7-{4-[(3-Carboxy-propyl)-carbamoyl] -1-piperazinyl} -1-cyclopropyl-6-fluor-1,4-dihydro-4-oxo- 3-chinolincarbonsäure mit einem Zersetzungspunkt von 245–248 °C.

Auf entsprechende Weise wie in Beispiel 38 werden folgende Verbindungen durch Hydrolyse der Produkte aus den Beispielen 35 und 36 erhalten:

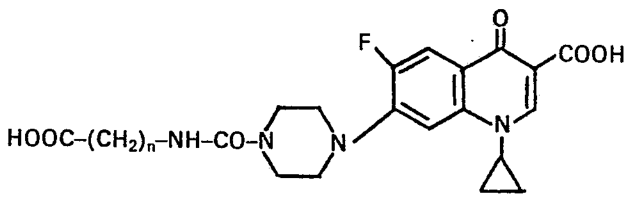

| Beispiel | n | Schmelzpunkt (°C) |
|---|---|---|
| 39 | 5 | 224 (Zers.) |
| 40 | 2 | 209 (Zers.) |

Beispiel 41

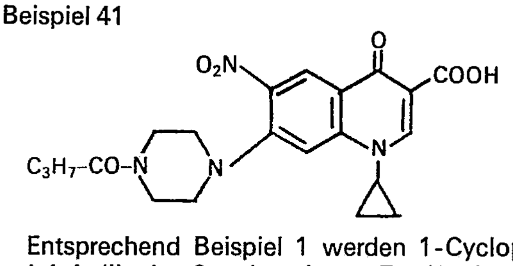

Entsprechend Beispiel 1 werden 1-Cyclopropyl-1,4-dihydro-6- nitro-4-oxo-7- (1-piperazinyl) -3-chinolincarbonsäure und Buttersäurechlorid zu 7-(4-Butyryl-1- piperazinyl) -1-cyclopropyl-1,4- dihydro-6-nitro-4-oxo -3-chinolincarbonsäure mit einem Zersetzungspunkt von 223–226 °C umgesetzt.

Beispiel 42

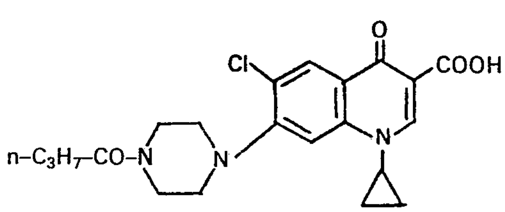

Entsprechend Beispiel 1 werden 6-Chlor-1-cyclopropyl-1,4- dihydro-4-oxo-7- (1-piperazinyl) -3-chinolincarbonsäure und Buttersäurechlorid zu 7-(4-Butyryl-1-piperazinyl) -6-chlor-1-cyclopropyl-1,4- dihydro-4-oxo-3- chinolincarbonsäure mit einem Zersetzungspunkt von 280–283 °C umgesetzt.

Beispiel 43

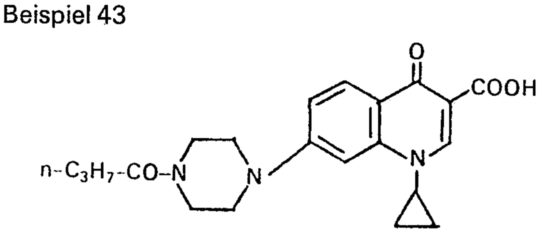

Entspredchend Beispiel 1 werden 1-Cyclopropyl-1,4-dihydro-4-oxo-7- (1-piperazinyl) -3-chinolincarbonsäure und Buttersäurechlorid zu 7-(4-Butyryl-1-piperazinyl) -1-cyclopropyl-1,4-dihydro-4-oxo- 3-chinolincarbonsäure mit einem Zersetzungspunkt von 252–255 °C umgesetzt.

Beispiel 44

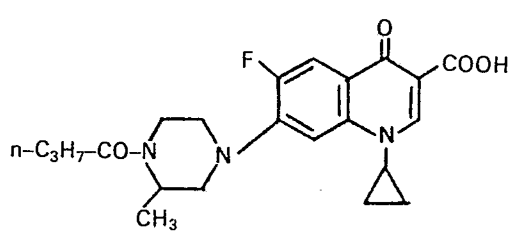

Entsprechend Beispiel 1 werden 1-Cyclopropyl-6-fluor-1,4- dihydro-4-oxo-7- (3-methyl-1-piperazinyl) -3-chinolincarbonsäure und Buttersäurechloird zu 7-(4-Butyryl-3-methyl-1- piperazinyl) -1-cyclopropyl-6-fluor-1,4- dihydro-4-oxo-3- chinolincarbonsäure mit einem Zersetzungspunkt von 226–228 °C umgesetzt.

Die erfindungsgemässen Verbindungen haben gute Wirkungen gegen grampositive und gramnegative Bakterien. In der nachstehenden Tabelle sind die minimalen Hemmkonzentrationen für erfindungsgemässe Verbindungen bei einigen Bakterien angegeben. Sie wurden im Falle des Beispiels 20 erhalten im Agarverdünnungstest mit Hilfe von Multipoint-Inokulator (Denley) auf Isosensitest-Agar, im Falle der Beispiele 17, 21, 15 mit dem Agarinkorporationstest auf DST-Medium.

| Stamm | Bsp. 17 | Bsp. 21 | Bsp. 20 | Bsp. 15 |
|---|---|---|---|---|
| E. coli Neumann | 0,03 | 0,06 | | 0,03 |
| Klebs. 8085 | 0,125 | | | 0,125 |
| Proteus 1017 | 0,03 | 0,06 | | 0,03 |
| Pseudom. W. | 0,5 | | | 0,5 |
| Staph. 133 | | 2 | 0,25 | 1 |

## Patentansprüche

1. Chinolincarbonsäuren der Formel (I)

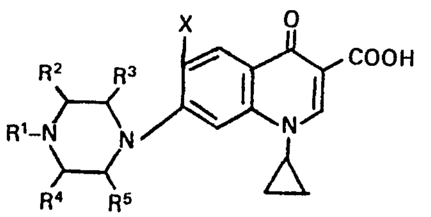

(I)

in der

$R^1$ für einen Rest $CO-R^6$, CN, $SO_2-R^7$ oder $S-R^8$ steht, wobei

$R^6$ Wasserstoff, gegebenenfalls durch 1 bis 3 Substituenten aus der Reihe Amino, Chlor, Alkoxycarbonyl mit 1 bis 4 Kohlenstoffatomen im Alkylteil, Carboxy, Alkoxy mit 1 bis 4 Kohlenstoffatomen, Hydroxy oder Trifluormethylthio substituiertes geradkettiges oder verzweigtes Alkyl mit 1 bis 5 C-Atomen, gegebenenfalls durch 1 bis 5 Substituenten aus der Reihe Fluor, Chlor, Hydroxy, Methoxy, Amino oder Carboxy substituiertes Phenyl, gegebenenfalls durch Amino, substituiertes Benzyl, gegebenenfalls durch Fluor, Chlor, Pyrazol-1-yl, 1,2,3-Triazol-1-yl, N-Oxido-2-, -3- oder -4-pyridylmethyl substituiertes Alkoxy oder Alkylthio mit 1 bis 5 C-Atomen, Benzyloxy, Amino, gegebenenfalls durch Alkoxycarbonyl mit 1 bis 3 C-Atomen im Alkylteil, Benzyloxycarbonyl oder Carboxy substituiertes Alkylamino mit 1 bis 5 C-Atomen, Phenylamino,

$R^7$ gegebenenfalls durch 1 bis 3 Substituenten aus der Reihe Fluor oder Amino substituiertes geradkettiges oder verzweigtes Alkyl mit 1 bis 4 C-Atomen, Phenyl, Methylphenyl,

$R^8$ Methoxycarbonyl, Trichlormethyl, Trifluormethyl oder Dichlorfluormethyl bedeuten,

$R^2$, $R^3$, $R^4$ und $R^5$ gleich oder verschieden sein können und für Wasserstoff, Methyl, Ethyl, n- oder i-Propyl stehen, und

X für Wasserstoff, Halogen oder Nitro steht, und deren pharmazeutisch verwendbare Säureadditions-, Alkali- und Erdalkalisalze und Hydrate.

2. Chinoloncarbonsäuren der Formel (I) in Anspruch I, in der

$R^1$ $CO-R^6$, CN, $SO_2-R^7$, $S-R^8$,

$R^6$ Wasserstoff, gegebenenfalls durch 1 bis 3 Substituenten aus der Reihe Amino, Chlor, Alkoxycarbonyl mit 1 bis 4 Kohlenstoffatomen im Alkylteil, Carboxy, Alkoxy mit 1 bis 4 Kohlenstoffatomen, Hydroxy oder Trifluormethylthio substituiertes geradkettiges oder verzweigtes Alkyl mit 1 bis 5 C-Atomen, gegebenenfalls durch 1 bis 5 Substituenten aus der Reihe Fluor, Chlor, Hydroxy, Methoxy, Amino oder Carboxy substituiertes Phenyl, gegebenenfalls durch Amino substituiertes Benzyl, gegebenenfalls durch Fluor, Chlor, Pyrazol-1-yl, 1,2,3-Triazol-1-yl, N-Oxido-2-, -3- oder -4-pyridylmethyl substituiertes Alkoxy oder Alkylthio mit 1 bis 5 C-Atomen, Benzyloxy, Amino, gegebenenfalls durch Alkoxycarbonyl mit 1 bis 3 C-Atomen im Alkylteil, Benzyloxycarbonyl oder Carboxy substituiertes Alkylamino mit 1 bis 5 C-Atomen, Phenylamino,

$R^7$ gegebenenfalls durch 1 bis 3 Substituenten aus der Reihe Fluor oder Amino substituiertes geradkettiges oder verzweigtes Alkyl mit 1 bis 4 C-Atomen, Phenyl, Methylphenyl,

$R^8$ Methoxycarbonyl, Trichlormethyl, Dichlorfluormethyl,

$R^2$, $R^3$, $R^4$ und $R^5$ Wasserstoff, Methyl oder Ethyl und

X Wasserstoff, Fluor, Chlor oder Nitro bedeuten.

3. Chinoloncarbonsäuren der Formel (I) in Anspruch 1, in der

$R^1$ $CO-R^6$, CN, $SO_2-R^7$, $S-R^8$,

$R^6$ Wasserstoff, gegebenenfalls durch 1 oder 2 Substituenten aus der Reihe Amino, Alkoxycarbonyl mit 1 bis 3 Kohlenstoffatomen im Alkylteil, Carboxy, Alkoxy mit 1 bis 3 Kohlenstoffatomen oder Trifluormethylthio substituiertes geradkettiges oder verzweigtes Alkyl mit 1 bis 4 C-Atomen, gegebenenfalls durch 1 bis 5 Substituenten aus der Reihe Chlor, Hydroxy, Amino oder Carboxy substituiertes Phenyl, gegebenenfalls durch Amino substituiertes Benzyl, gegebenenfalls durch Pyrazol-1-yl, 1,2,3-Triazol-1-yl, N-Oxi-

do-2-, -3- oder -4-pyridylmethyl substituiertes Alkoxy mit 1 bis 4 C-Atomen, Alkylthio mit 1 bis 2 C-Atomen, Benzyloxy, Amino, gegebenenfalls durch Alkoxycarbonyl mit 1 bis 3 C-Atomen im Alkylteil oder Carboxy substituiertes Alkylamino mit 1 bis 5 C-Atomen,

$R^7$ geradkettiges oder vezweigtes Alkyl mit 1 bis 3 C-Atomen, Phenyl, Methylphenyl,

$R^8$ Methoxycarbonyl, Trichlormethyl, Dichlorfluormethyl,

$R^2$ Wasserstoff, Methyl, Ethyl,

$R^3$ Wasserstoff,

$R^4$ Wasserstoff, Methyl,

$R^5$ Wasserstoff, und

X Wasserstoff, Fluor, Chlor oder Nitro bedeuten.

4. 7-[4-(Dichlorfluormethylsulfonyl)-1-piperazinyl]-1-cyclopropyl- 6-fluor-1,4-dihydro-4-oxo-3-chinolincarbonsäure.

5. 7-[4-(Dichlorfluormethylsulfenyl)-1-piperazinyl]- 1-cyclopropyl-6-fluor-1,4-dihydro-4-oxo-3-chinolincarbonsäure.

6. 1-Cyclopropyl-6-fluor-7-(4-formyl-1-piperazinyl)- 1,4-dihydro-4-oxo-3-chinolincarbonsäure.

7. Chinoloncarbonsäuren der Formel (I)

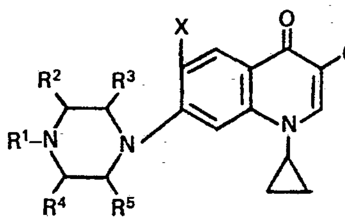

(I)

in der

$R^1$ für einen Rest $CO-R^6$, CN, $SO_2-R^7$ oder $S-R^8$ steht, wobei

$R^6$ Wasserstoff, gegebenenfalls durch 1 bis 3 Substituenten aus der Reihe Amino, Chlor, Alkoxycarbonyl mit 1 bis 4 Kohlenstoffatomen im Alkylteil, Carboxy, Alkoxy mit 1 bis 4 Kohlenstoffatomen, Hydroxy oder Trifluormethylthio substituiertes geradkettiges oder verzweigtes Alkyl mit 1 bis 5 C-Atomen, gegebenenfalls durch 1 bis 5 Substituenten aus der Reihe Fluor, Chlor, Hydroxy, Methoxy, Amino oder Carboxy substituiertes Phenyl, gegebenenfalls durch Amino substituiertes Benzyl, gegebenenfalls durch Fluor, Chlor, Pyrazol-1-yl, 1,2,3-Triazol-1-yl, N-Oxido-2-, -3- oder -4-pyridylmethyl substituiertes Alkoxy oder Alkylthio mit 1 bis 5 C-Atomen, Benzyloxy, Amino, gegebenenfalls durch Alkoxycarbonyl mit 1 bis 3 C-Atomen im Alkylteil, Benzyloxycarbonyl oder Carboxy substituiertes Alkylamino mit 1 bis 5 C-Atomen, Phenylamino,

$R^7$ gegebenenfalls durch 1 bis 3 Substituenten aus der Reihe Fluor oder Amino substituiertes geradkettiges oder verzweigtes Alkyl mit 1 bis 4 C-Atomen, Phenyl, Methylphenyl,

$R^8$ Methoxycarbonyl, Trichlormethyl, Trifluormethyl oder Dichlorfluormethyl bedeuten,

$R^2$, $R^3$, $R^4$ und $R^5$ gleich oder verschieden sein können und für Wasserstoff, Methyl, Ethyl, n- oder i-Propyl stehen, und

X für Wasserstoff, Halogen oder Nitro steht, und deren pharmazeutisch verwendabre Säureadditions-, Alkali- und Erdalkalisalze und Hydrate, zur Anwendung in einem Verfahren zur therapeutischen Behandlung des menschlichen oder tierischen Körpers.

8. Verfahren zur Herstellung von Chinoloncarbonsäuren der Formel (I)

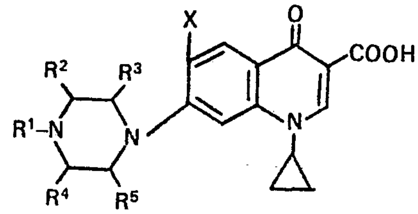

(I)

in der

$R^1$ für einen Rest $CO-R^6$, CN, $SO_2-R^7$ oder $S-R^8$ steht, wobei

$R^6$ Wasserstoff, gegebenenfalls durch 1 bis 3 Substituenten aus der Reihe Amino, Chlor, Alkoxycarbonyl mit 1 bis 4 Kohlenstoffatomen im Alkylteil, Carboxy, Alkoxy mit 1 bis 4 Kohlenstoffatomen, Hydroxy oder Trifluormethylthio substituiertes geradkettiges oder verzweigtes Alkyl mit 1 bis 5 C-Atomen, gegebenenfalls durch 1 bis 5 Substituenten aus der Reihe Fluor, Chlor, Hydroxy, Methoxy, Amino oder Carboxy substituiertes Phenyl, gegebenenfalls durch Amino substituiertes Benzyl, gegebenenfalls durch Fluor, Chlor, Pyrazol-1-yl, 1,2,3-Triazol-1-yl, N-Oxido-2-, -3- oder -4-pyridylmethyl substituiertes Alkoxy oder Alkylthio mit 1 bis 5 C-Atomen, Benzyloxy, Amino, gegebenenfalls durch Alkoxycarbonyl mit 1 bis 3 C-Atomen im Alkylteil, Benzyloxycarbonyl oder Carboxy substituiertes Alkylamino mit 1 bis 5 C-Atomen, Phenylamino,

$R^7$ gegebenenfalls durch 1 bis 3 Substituenten aus der Reihe Fluor oder Amino substituiertes geradkettiges oder verzweigtes Alkyl mit 1 bis 4 C-Atomen, Phenyl, Methylphenyl,

$R^8$ Methoxycarbonyl, Trichlormethyl, Trifluormethyl oder Dichlorfluormethyl bedeuten,

$R^2$, $R^3$, $R^4$ und $R^5$ gleich oder verschieden sein können und für Wasserstoff, Methyl, Ethyl, n- oder i-Propyl stehen, und

X für Wasserstoff, Halogen oder Nitro steht, und deren pharmazeutisch verwendbare Säureadditions-, Alkali- und Erdalkalisalze und Hydrate, dadurch gekennzeichnet, dass man entweder

a) Verbindungen der Formel (II)

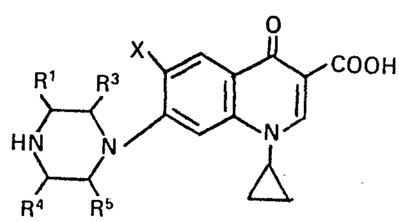

(II)

in welcher

X, $R^2$, $R^3$, $R^4$ und $R^5$ die oben angegebenen Bedeutungen haben, mit einer Verbindung der Formel (III)

$R^1$–Y (III)

in welcher

$R^1$ die oben angegebene Bedeutung hat und

Y für eine Abgangsgruppe, wie Halogen, Methoxy, Ethoxy, Phenoxy, 4-Nitrophenoxy oder 2,3,5-Trichlorphenoxy, Alkoxycarbonyloxy, steht, umsetzt, oder dass man

b) Verbindungen der Formel (II)

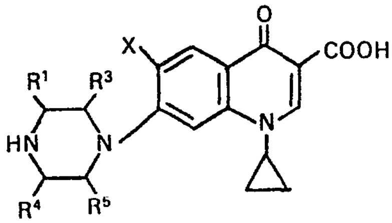

(II)

mit Isocyanaten der Formel (IV)

R'–NCO (IV)

in welcher

R' gegebenenfalls substituiertes Alkyl oder Phenyl bedeutet, umsetzt, oder dass man

c) Verbindungen der Formel (II)

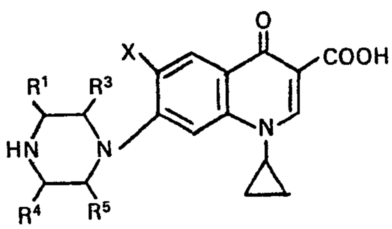

(II)

mit Anhydriden der Formel (V)

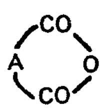

(V),

in welcher

A eine gegebenenfalls substituierte Alkylenkette mit 2 oder 3 Kohlenstoffatomen oder einen Arylenrest bedeutet, umsetzt.

9. Arzneimittel, gekennzeichnet durch einen Gehalt an mindestens einer Chinoloncarbonsäure der Formel (I) in Anspruch 1.

10. Verwendung von Chinoloncarbonsäuren der Formel (I)

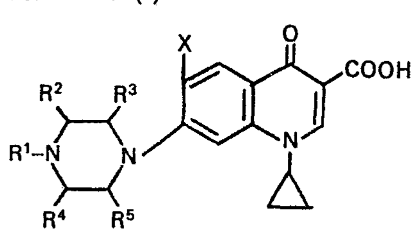

(I)

in der

$R^1$ für einen Rest CO–$R^6$, CN, $SO_2$–$R^7$ oder S–$R^8$ steht, wobei

$R^6$ Wasserstoff, gegebenenfalls durch 1 bis 3 Substituenten aus der Reihe Amino, Chlor, Alkoxycarbonyl mit 1 bis 4 Kohlenstoffatomen im Alkylteil, Carboxy, Alkoxy mit 1 bis 4 Kohlenstoffatomen, Hydroxy oder Trifluormethylthio substituiertes geradkettiges oder verzweigtes Alkyl mit 1 bis 5 C-Atomen, gegebenenfalls durch 1 bis 5 Substituenten aus der Reihe Fluor, Chlor, Hydroxy, Methoxy, Amino oder Carboxy substituiertes Phenyl, gegebenenfalls durch Amino substituiertes Benzyl, gegebenenfalls durch Fluor, Chlor, Pyrazol-1-yl, 1,2,3-Triazol-1-yl, N-Oxido-2-, -3- oder -4-pyridylmethyl substituiertes Alkoxy oder Alkylthio mit 1 bis 5 C-Atomen, Benzyloxy, Amino, gegebenenfalls durch Alkoxycarbonyl mit 1 bis 3 C-Atomen im Alkylteil, Benzyloxycarbonyl oder Carboxy substituiertes Alkylamino mit 1 bis 5 C-Atomen, Phenylamino,

$R^7$ gegebenenfalls durch 1 bis 3 Substituenten aus der Reihe Fluor oder Amino substituiertes geradkettiges oder verzweigtes Alkyl mit 1 bis 4 C-Atomen, Phenyl, Methylphenyl,

$R^8$ Methoxycarbonyl, Trichlormethyl, Trifluormethyl oder Dichlorfluormethyl bedeuten,

$R^2$, $R^3$, $R^4$ und $R^5$ gleich oder verschieden sein können und für Wasserstoff, Methyl, Ethyl, n- oder i-Propyl stehen, und

X für Wasserstoff, Halogen oder Nitro steht, und deren pharmazeutisch verwendbaren Säureadditions-, Alkali- und Erdalkalisalzen und Hydraten, zur Herstellung von Arzneimitteln.

## Revendications

1. Acides quinolonecarboxyliques de formule (I)

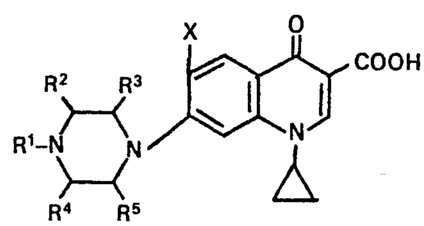

(I)

dans laquelle

$R^1$ représente un reste CO–$R^6$, CN, $SO_2$–$R^7$ ou S–$R^8$, où

$R^6$ représente l'hydrogène, un groupe alkyle en $C_1$ à $C_5$ à chaîne droite ou ramifié éventuellement substitué par 1 à 3 substituants choisis dans la série amino, chloro, alkoxycarbonyle ayant 1 à 4 atomes de carbone dans la partie alkyle, carboxy, alkoxy ayant 1 à 4 atomes de carbone, hydroxy ou trifluorométhylthio, phényle éventuellement substitué par 1 à 5 substituants de la série fluoro, chloro, hydroxy, méthoxy, amino ou carboxy, benzyle éventuellement substitué par un radical amino, alkoxy ou alkylthio en $C_1$ à $C_5$ éventuellement substitué par un radical fluoro, chloro, pyrazole-1-yle, 1,2,3-triazole-1-yle, N-oxydo-2-, -3- ou -4-pyridylméthyle, un groupe benzyloxy, amino, alkylamino en $C_1$ à $C_5$ éventuellement

substitué par un radical alkoxycarbonyle ayant 1 à 3 atomes de carbone dans la partie alkyle, benzyloxycarbonyle ou carboxy, un groupe phénylamino,

$R^7$ désigne un groupe alkyle en $C_1$ à $C_4$ à chaîne droite ou ramifié éventuellement substitué par 1 à 3 substituants de la série fluoro ou amino, un groupe phényle, méthylphényle,

$R^8$ est un groupe méthoxycarbonyle, trichlorométhyle, trifluorométhyle ou dichlorofluorométhyle,

$R^2$, $R^3$, $R^4$ et $R^5$ peuvent être identiques ou différents et représentent l'hydrogène ou un radical méthyle, éthyle, n-propyle ou isopropyle, et

X désigne l'hydrogène, un halogène ou un groupe nitro, et leurs sels d'addition d'acides, leurs sels alcalins et leurs sels alcalino-terreux utilisables en pharmacie.

2. Acides quinolonecarboxyliques de formule (I) suivant la revendication 1, dans laquelle

$R^1$ représente $CO-R^6$, CN, $SO_2-R^7$, $S-R^8$,

$R^6$ représente l'hydrogène, un groupe alkyle en $C_1$ à $C_5$ à chaîne droite ou ramifié éventuellement substitué par 1 à 3 substituants de la série amino, chloro, alkoxycarbonyle ayant 1 à 4 atomes de carbone dans la partie alkyle, carboxy, alkoxy ayant 1 à 4 atomes de carbone, hydroxy ou trifluorométhylthio, un groupe phényle éventuellement substitué par 1 à 5 substituants de la série fluoro, chloro, hydroxy, méthoxy, amino ou carboxy, un groupe benzyle éventuellement substitué par un radical amino, un groupe alkoxy ou alkylthio en $C_1$ à $C_5$ éventuellement substitué par un radical fluoro, chloro, pyrazole-1-yle, 1,2,3-triazole-1-yle, N-oxydo-2-, -3- ou -4-pyridylméthyle, un groupe benzyloxy, amino, alkylamino en $C_1$ à $C_5$ éventuellement substitué par un radical alkoxycarbonyle ayant 1 à 3 atomes de carbone dans la partie alkyle, benzyloxycarbonyle ou carboxy, un groupe phénylamino,

$R^7$ est un groupe alkyle à chaîne droite ou ramifié ayant 1 à 4 atomes de carbone, éventuellement substitué par 1 à 3 substituants de la série fluoro ou amino, un groupe phényle, méthylphényle,

$R^8$ est un groupe méthoxycarbonyle, trichlorométhyle, dichlorofluorométhyle,

$R^2$, $R^3$, $R^4$ et $R^5$ représentent l'hydrogène, le groupe méthyle ou le groupe éthyle et

X est l'hydrogène, le fluor, le chlore ou le groupe nitro.

3. Acides quinolonecarboxyliques de formule (I) suivant la revendication 1, dans laquelle

$R^1$ est un groupe $CO-R^6$, CN, $SO_2-R^7$, $S-R^8$,

$R^6$ représente l'hydrogène, un groupe alkyle $C_1$ à $C_4$ à chaîne droite ou ramifié éventuellement substitué par 1 ou 2 substituants de la série amino, alkoxycarbonyle ayant 1 à 3 atomes de carbone dans la partie alkyle, carboxy, alkoxy ayant 1 à 3 atomes de carbone ou trifluorométhylthio, un groupe phényle éventuellement substitué par 1 à 5 substituants de la série chloro, hydroxy, amino ou carboxy, un groupe benzyle éventuellement substitué par un radical amino, un groupe alkoxy en $C_1$ à $C_4$ éventuellement substitué par un radical pyrazole-1-yle, 1,2,3-triazole-1-yle, N-oxydo-2-, -3- ou -4-pyridylméthyle, un groupe alkylthio ayant 1 ou 2 atomes de carbone, benzyloxy, amino, un groupe alkylamino en $C_1$ à $C_5$ éventuellement substitué par un radical alkoxycarbonyle ayant 1 à 3 atomes de carbone dans la partie alkyle ou un radical carboxy,

$R^7$ est un groupe alkyle à chaîne droite ou ramifié ayant 1 à 3 atomes de carbone, phényle, méthylphényle,

$R^8$ est un groupe méthoxycarbonyle, trichlorométhyle, dichlorofluorométhyle,

$R^2$ est l'hydrogène, un groupe méthyle, éthyle,

$R^3$ est l'hydrogène,

$R^4$ est l'hydrogène, un groupe méthyle,

$R^5$ est l'hydrogène, et

X est l'hydrogène, le fluor, le chlore ou un groupe nitro.

4. L'acide 7-[4-(dichlorofluorométhylsulfonyl)- 1-pipérazinyl]-1- cyclopropyl-6- fluoro-1,4- dihydro-4- oxo -3-quinoléine-carboxylique.

5. L'acide 7-[4- (dichlorofluorométhylsulfényl)- 1- pipérazinyl]- 1-cyclopropyl-6- fluoro-1,4-dihydro-4-oxo-3-quinoléine-carboxylique.

6. L'acide 1-cyclopropyl-6- fluoro-7- (4-formyl-1- pipérazinyl)- 1,4- dihydro-4- oxo-3-quinoléine-carboxylique.

7. Acides quinolonecarboxyliques de formule (I)

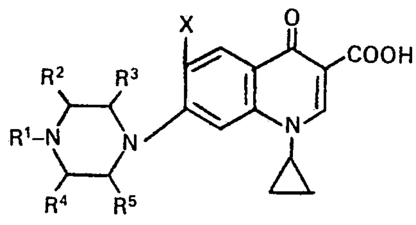

(I)

dans laquelle

$R^1$ est un reste $CO-R^6$, CN, $SO_2-R^7$ ou $S-R^8$, où

$R^6$ représente l'hydrogène, un groupe alkyle à chaîne droite ou ramifié en $C_1$ à $C_5$ éventuellement substitué par 1 à 3 substituants de la série amino, chloro, alkoxycarbonyle ayant 1 à 4 atomes de carbone dans la partie alkyle, carboxy, alkoxy ayant 1 à 4 atomes de carbone, hydroxy ou trifluorométhylthio, un groupe phényle éventuellement substitué par 1 à 5 substituants de la série fluoro, chloro, hydroxy, méthoxy, amino ou carboxy, un groupe benzyle éventuellement substitué par un radical amino, un groupe alkoxy ou alkylthio en $C_1$ à $C_5$ éventuellement substitué par un radical fluoro, chloro, pyrazole-1-yle, 1,2,3-triazole-1-yle, N-oxydo-2-, -3- ou -4-pyridylméthyle, un groupe benzyloxy, amino, alkylamino en $C_1$ à $C_5$ éventuellement substitué par un radical alkoxycarbonyle ayant 1 à 3 atomes de carbone dans la partie alkyle, benzyloxycarbonyle ou carboxy, un groupe phénylamino,

$R^7$ est un groupe alkyle en $C_1$ à $C_4$ à chaîne droite ou ramifié éventuellement substitué par 1 à 3 substituants de la série fluoro ou amino, un groupe phényle, méthylphényle,

$R^8$ est un groupe méthoxycarbonyle, trichloro-

méthyle, trifluorométhyle ou dichlorofluorométhyle,

$R^2$, $R^3$, $R^4$ et $R^5$ peuvent être identiques ou différents et représentent l'hydrogène, un groupe méthyle, éthyle, n-propyle ou isopropyle, et

X est l'hydrogène, un halogène ou un groupe nitro, et leurs sels d'addition d'acides, leurs sels alcalins et leurs sels alcalinoterreux et leurs hydrates pharmaceutiquement acceptables, destinés à être utilisés dans un procédé pour le traitement thérapeutique du corps humain ou d'animaux.

8. Procédé de production d'acides quinolonecarboxyliques de formule (I)

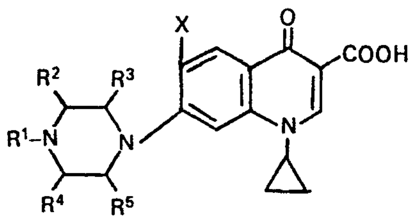

(I)

dans laquelle

$R^1$ représente un reste $CO-R^6$, CN, $SO_2-R^7$ ou $S-R^8$, où

$R^6$ représente l'hydrogène, alkyle en $C_1$ à $C_5$ à chaîne droite ou ramifié éventuellement substitué par 1 à 3 substituants choisis dans la série amino, chloro, alkoxycarbonyle ayant 1 à 4 atomes de carbone dans la partie alkyle, carboxy, alkoxy ayant 1 à 4 atomes de carbone, hydroxy ou trifluorométhylthio, phényle éventuellement substitué par 1 à 5 substituants de la série fluoro, chloro, hydroxy, méthoxy, amino ou carboxy, benzyle éventuellement substitué par un radical amino, alkoxy ou alkylthio en $C_1$ à $C_5$ éventuellement substitué par un radical fluoro, chloro, pyrazole-1-yle,1,2,3-triazole-1-yle, N-oxydo-2-, -3- ou -4-pyridylméthyle, un groupe benzyloxy, amino, alkylamino en $C_1$ à $C_5$ éventuellement substitué par un radical alkoxycarbonyle ayant 1 à 3 atomes de carbone dans la partie alkyle, benzyloxycarbonyle ou carboxy, un groupe phénylamino,

$R^7$ désigne un groupe alkyle en $C_1$ à $C_4$ à chaîne droite ou ramifié éventuellement substitué par 1 à 3 substituants de la série fluoro ou amino, un groupe phényle, méthylphényle,

$R^8$ est un groupe méthoxycarbonyle, trichlorométhyle, trifluorométhyle ou dichlorofluorométhyle,

$R^2$, $R^3$, $R^4$ et $R^5$ peuvent être identiques ou différents et représentent l'hydrogène ou un radical méthyle, éthyle, n-propyle ou isopropyle, et

X désigne l'hydrogène, un halogène ou un groupe nitro, et de leurs sels d'addition d'acides, leurs sels alcalins et alcalinoterreux et leurs hydrates acceptables en pharmacie, caractérisé en ce que:

a) on fait réagir des composés de formule (II)

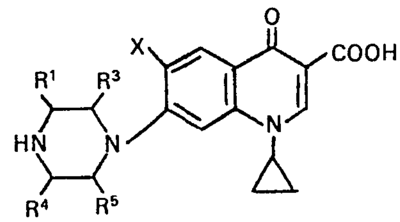

(II)

dans laquelle

X, $R^2$, $R^3$, $R^4$ et $R^5$ ont les définitions indiquées ci-dessus, avec un composé de formule (III)

$R^1-Y$ (III)

dans laquelle

$R^1$ a la définition indiquée ci-dessus et

Y est un groupe partant tel qu'un halogène, un groupe méthoxy, éthoxy, phénoxy, 4-nitrophénoxy ou 2,3,5-trichlorophénoxy, alkoxycarbonyloxy, ou bien

b) on fait réagir des composés de formule (II)

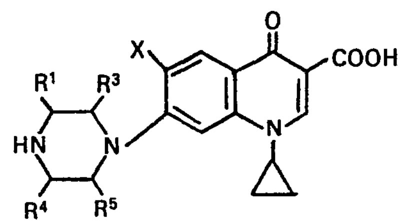

(II)

avec des isocyanates de formule (IV)

$R'-NCO$ (IV)

dans laquelle

R' désigne un groupe alkyle ou phényle éventuellement substitué, ou bien

c) on fait réagir des composés de formule (II)

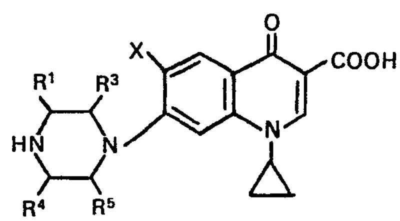

(II)

avec des anhydrides de formule (V)

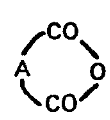

(V),

dans laquelle

A est une chaîne alkylénique de 2 ou 3 atomes de carbone éventuellement substituée ou un reste arylène.

9. Médicament, caractérisé par une teneur en au moins un acide quinolonecarboxylique de formule (I) suivant la revendication 1.

10. Utilisation d'acides quinolonecarboxyliques de formule (I)

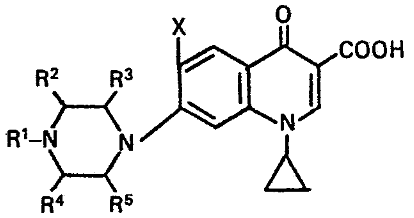

(I)

dans laquelle

$R^1$ représente un reste $CO-R^6$, CN, $SO_2-R^7$ ou $S-R^8$, où

$R^6$ représente l'hydrogène, un groupe alkyle en $C_1$ à $C_5$ à chaîne droite ou ramifié éventuellement substitué par 1 à 3 substituants choisis dans la série amino, chloro, alkoxycarbonyle ayant 1 á 4 atomes de carbone dans la partie alkyle, carboxy, alkoxy ayant 1 à 4 atomes de carbone, hydroxy ou trifluorométhylthio, phényle éventuellement substitué par 1 à 5 substituants de la série fluoro, chloro, hydroxy, méthoxy, amino ou carboxy, benzyle éventuellement substitué par un radical amino, alkoxy ou alkylthio en $C_1$ à $C_5$ éventuellement substitué par un radical fluoro, chloro, pyrazole-1-yle, 1,2,3-triazole-1-yle, N-oxydo-2-, -3- ou -4-pyridylméthyle, un groupe benzyloxy, amino, alkylamino en $C_1$ à $C_5$ éventuellement substitué par un radical alkoxycarbonyle ayant 1 à 3 atomes de carbone dans la partie alkyle, benzyloxycarbonyle ou carboxy, un groupe phénylamino,

$R^7$ désigne un groupe alkyle en $C_1$ à $C_4$ à chaîne droite ou ramifié éventuellement substitué par 1 à 3 substituants de la série fluoro ou amino, un groupe phényle, méthylphényle,

$R^8$ est un groupe méthoxycarbonyle, trichlorométhyle, trifluorométhyle ou dichlorofluorométhyle,

$R^2$, $R^3$, $R^4$ et $R^5$ peuvent être identique ou différents et représentent l'hydrogène ou un radical méthyle, éthyle, n-propyle ou isopropyle, et

X désigne l'hydrogène, un halogène ou un groupe nitro, et de leurs sels d'addition d'acides, de leurs sels alcalins et alcalinoterreux et leurs hydrates pharmaceutiquement acceptables pour la préparation de médicaments.

## Claims

1. Quinolonecarboxylic acids of the formula (I)

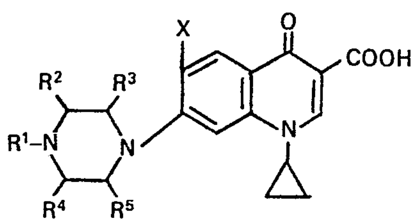

(I)

in which

$R^1$ represents a radical $CO-R^6$, CN, $SO_2-R^7$ oder $S-R^8$, wherein

$R^6$ denotes hydrogen, straigt-chain or branched alkyl which has 1 to 5 C atoms and is optionally substituted by 1 to 3 substituents from the series comprising amino, chlorine, alkoxycarbonyl with 1 to 4 carbon atoms in the alkyl part, carboxyl, alkoxy with 1 to 4 carbon atoms, hydroxyl and trifluoromethylthio, phenyl which is optionally substituted by 1 to 5 substituents from the series comprising fluorine, chlorine, hydroxyl, methoxy, amino and carboxyl, benzyl which is opitonally substituted by amino, alkoxy or alkylthio which has 1 to 5 C atoms and is optionally substituted by fluorine, chlorine, pyrazol-1-yl, 1,2,3-triazol-1-yl, or N-oxido-2-, -3- or -4-pyridylmethyl, benzyloxy, amino, alkylamino which has 1 to 5 C atoms and is optionally substituted by alkoxycarbonyl with 1 to 3 C atoms in the alkyl part, benzyloxycarbonyl or carboxyl, or phenylamino,

$R^7$ denotes straight-chain or branched alkyl which has 1 to 4 C atoms and is optionally substituted by 1 to 3 substitutents from the series comprising fluorine and amino, phenyl, or methylphenyl,

$R^8$ denotes methoxycarbonyl, trichloromethyl, trifluoromethyl or dichlorofluoromethyl,

$R^2$, $R^3$, $R^4$ and $R^5$ can be identical or different and represent hydrogen, methyl, ethyl, n- or i-propyl, and

X represents hydrogen, halogen or nitro, and their pharmaceutically usable acid addition, alkali metal and alkaline earth metal salts and hydrates.

2. Quinolonecarboxylic acids of the formula (I) in Claim 1, in which

$R^1$ denotes $CO-R^6$, CN, $SO_2-R^7$ or $S-R^8$,

$R^6$ denotes hydrogen, straight-chain or branched alkyl which has 1 to 5 C atoms and is optionally substituted by 1 to 3 substituents from the series comprising amino, chlorine, alkoxycarbonyl with 1 to 4 carbon atoms in the alkyl part, carboxyl, alkoxy with 1 to 4 carbon atoms, hydroxyl and trifluoromethylthio, phenyl which is optionally substituted by 1 to 5 substituents from the series comprising fluorine, chlorine, hydroxyl, methoxy, amino and carboxyl, benzyl which is optionally substituted by amino, alkoxy or alkylthio which has 1 to 5 C atoms and is optionally substituted by fluorine, chlorine, pyrazol-1-yl, 1,2,3-triazol-1-yl, or N-oxido-2-, -3- or -4-pyridylmethyl, benzyloxy, amino, alkylamino which has 1 to 5 C atoms and is optionally substituted by alkoxycarbonyl with 1 to 3 C atoms in the alkyl part, benzyloxycarbonyl or carboxyl, or phenylamino,

$R^7$ denotes straight-chain or branched alkyl which has 1 to 4 C atoms and is optionally substituted by 1 to 3 substituents from the series comprising fluorine and amino, phenyl, or methylphenyl,

$R^8$ denotes methoxycarbonyl, trichloromethyl, or dichlorofluoromethyl,

$R^2$, $R^3$, $R^4$ and $R^5$ denote hydrogen, methyl or ethyl and

X denotes hydrogen, fluorine, chlorine or nitro.

3. Quinolonecarboxylic acids of the formula (I) in Claim 1, in which

$R^1$ denotes $CO-R^6$, CN, $SO_2-R^7$ or $S-R^8$,

$R^6$ denotes hydrogen, straight-chain or branched alkyl which has 1 to 4 C atoms and is optionally substituted by 1 or 2 substituents from the series comprising amino, alkoxycarbonyl with 1 to 3 carbon atoms in the alkyl part, carboxyl, alkoxy with 1 to 3 carbon atoms and trifluoromethylthio, phenyl which is optionally substituted by 1 to 5 substituents from the series comprising chlorine, hydroxyl, amino and carboxyl, benzyl which is optionally substituted by amino, alkoxy which has 1 to 4 C atoms and is optionally substituted by pyrazol-1-yl, 1,2,3-triazol-1-yl, or N-oxido-2-, -3- or -4-pyridylmethyl, alkylthio with 1 to 2 C atoms, benzyloxy, amino, or alkylamino which has 1 to 5 C atoms and is optionally substituted by alkoxycarbonyl with 1 to 3 C atoms in the alkyl part or carboxyl,

$R^7$ denotes straight-chain or branched alkyl with 1 to 3 C atoms, phenyl, or methylphenyl,

$R^8$ denotes methoxycarbonyl, trichloromethyl, or dichloro-fluoromethyl,

$R^2$ denotes hydrogen, methyl or ethyl,

$R^3$ denotes hydrogen,

$R^4$ denotes hydrogen or methyl,

$R^5$ denotes hydrogen and

X denotes hydrogen, fluorine, chlorine or nitro.

4. 7-[4- (Dichlorofluoromethylsulphonyl)-1-piperazinyl]-1- cyclopropyl-6-fluoro-1,4-dihydro- 4-oxo-3-quinolinecarboxylic acid.

5. 7-[4- (Dichlorofluoromethylsulphenyl)-1-piperazinyl]- 1-cyclopropyl-6-fluoro-1,4-dihydro-4-oxo-3-quinolinecarboxylic acid.

6. 1-Cyclopropyl-6- fluoro-7- (4-formyl-1-piperazinyl)- 1,4-dihydro-4-oxo-3-quinolinecarboxylic acid.

7. Quinolonecarboxylic acids of the formula (I)

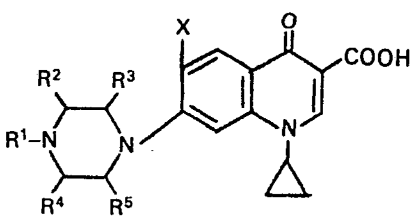

in which

$R^1$ represents a radical $CO-R^6$, CN, $SO_2-R^7$ or $S-R^8$, wherein

$R^6$ denotes hydrogen, straight-chain or branched alkyl which has 1 to 5 C atoms and is optionally substituted by 1 to 3 substituents from the series comprising amino, chlorine, alkoxycarbonyl with 1 to 4 carbon atoms in the alkyl part, carboxyl, alkoxy with 1 to 4 carbon atoms, hydroxyl and trifluoromethylthio, phenyl which is optionally substituted by 1 to 5 substituents from the series comprising fluorine, chlorine, hydroxyl, methoxy, amino and carboxyl, benzyl which is optionally substituted by amino, alkoxy or alkylthio which has 1 to 5 C atoms and is optionally substituted by fluorine, chlorine, pyrazol-1-yl, 1,2,3-triazol-1-yl, or N-oxido-2-, -3- or -4-pyridylmethyl, benzyloxy, amino, alkylamino which has 1 to 5 C atoms and is optionally substituted by alkoxycarbonyl with 1 to 3 C atoms in the alkyl part, benzyloxycarbonyl or carboxyl, or phenylamino,

$R^7$ denotes straight-chain or branched alkyl which has 1 to 4 C atoms and is optionally substituted by 1 to 3 substituents from the series comprising fluorine and amino, phenyl, or methylphenyl,

$R^8$ denotes methoxycarbonyl, trichloromethyl, trifluoromethyl or dichlorofluoromethyl,

$R^2$, $R^3$, $R^4$ and $R^5$ can be identical or different and represent hydrogen, methyl, ethyl, n- or i-propyl, and X represents hydrogen, halogen or nitro, and their pharmaceutically usable acid addition, alkali metal and alkaline earth metal salts and hydrates, for use in a porcess for the therapeutic treatment of the human or animal body.

8. Process for the preparation of quinolonecarboxylic acids of the formula (I)

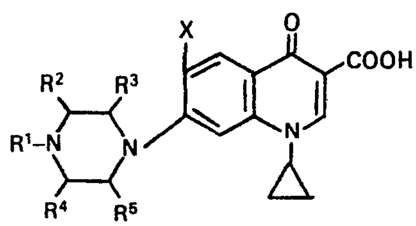

(I)

in which

$R^1$ represents a radical $CO-R^6$, CN, $SO_2-R^7$ or $S-R^8$, wherein

$R^6$ denotes hydrogen, straight-chain or branched alkyl which has 1 to 5 C atoms and is optionally substituted by 1 to 3 substituents from the series comprising amino, chlorine, alkoxycarbonyl with 1 to 4 carbon atoms in the alkyl part, carboxyl, alkoxy with 1 to 4 carbon atoms, hydroxyl and trifluoromethylthio, phenyl which is optionally substituted by 1 to 5 substituents from the series comprising fluorine, chlorine, hydroxyl, methoxy, amino and carboxyl, benzyl which is optionally substituted by amino, alkoxy or alkylthio which has 1 to 5 C atoms and is optionally substituted by fluorine, chlorine, pyrazol-1-yl, 1,2,3-triazol-1-yl, or N-oxido-2-, -3- or -4-pyridylmethyl, benzyloxy, amino, alkylamino which has 1 to 5 C atoms and is optionally substituted by alkoxycarbonyl with 1 to 3 C atoms in the alkyl part, benzyloxycarbonyl or carboxyl, or phenylamino,

$R^7$ denotes straight-chain or branched alkyl which has 1 to 4 C atoms and is optionally substituted by 1 to 3 substituents from the series comprising fluorine and amino, phenyl or methylphenyl,

$R^8$ denotes methoxycarbonyl, trichloromethyl, trifluoromethyl or dichlorofluoromethyl,

$R^2$, $R^3$, $R^4$ and $R^5$ can be identical or different and represent hydrogen, methyl, ethyl, n- or i-propyl, and X represents hydrogen, halogen or nitro, and their pharmaceutically usable acid addition, alkaly metal and alkaline earth metal salts and hydrates, characterised in that either

a) compounds of the formula (II)

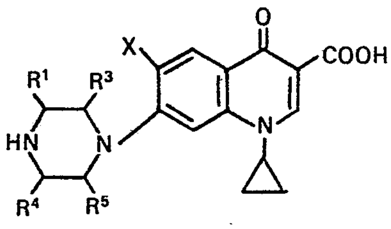

(II)

in which

X, $R^2$, $R^3$, $R^4$ and $R^5$ have the abovementioned meanings, are reacted with a compound of the formula

$R^1$–Y (III)

in which

$R^1$ had the abovementioned meaning and

Y represents a leaving group, such as halogen, methoxy, ethoxy, phenoxy, 4-nitrophenoxy or 2,3,5-trichlorophenoxy or alkoxycarbonyloxy, or

b) compounds of the formula (II)

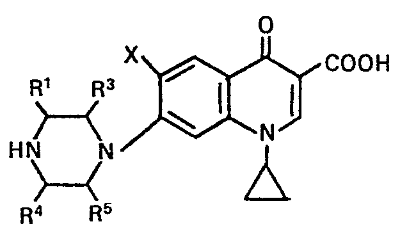

(II)

are reacted with isocyanates of the formula (IV)

R′–NCO (IV)

in which

R′ denotes optionally substituted alkyl or phenyl, or

c) compounds of the formula (II)

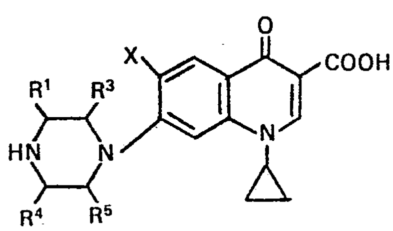

(II)

are reacted with anhydrides of the formula (V)

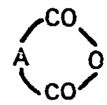

(V),

in which

A denotes an optionally substituted alkylene chain with 2 or 3 carbon atoms or an arylene radical.

9. Medicaments, characterised in that they contain at least one quinolonecarboxylic acid of the formula (I) in Claim 1.

10. Use of quinolonecarboxylic acids of the formula (I)

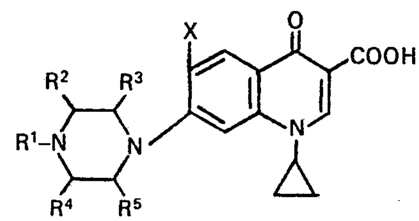

(I)

in which

$R^1$ represents a radical CO–$R^6$, CN, $SO_2$–$R^7$ or S–$R^8$, wherein

$R^6$ denotes hydrogen, straight-chain or branched alkyl which has 1 to 5 C atoms and is optionally substituted by 1 to 3 substituents from the series comprising amino, chlorine, alkoxycarbonyl with 1 to 4 carbon atoms in the alkyl part, carboxyl, alkoxy with 1 to 4 carbon atoms, hydroxyl and trifluoromethylthio, phenyl which is optionally substituted by 1 to 5 substituents from the series comprising fluorine, chlorine, hydroxyl, methoxy, amino and carboxyl, benzyl which is optionally substituted by amino, alkoxy or alkylthio which has 1 to 5 C atoms and is optionally substituted by fluorine, chlorine, pyrazol-1-yl, 1,2,3-triyzol-1-yl, or N-oxido-2-, -3- or -4-pyridylmethyl, benzyloxy, amino, alkylamino which has 1 to 5 C atoms and is optionally substituted by alkoxycarbonyl with 1 to 3 C atoms in the alkyl part, benzyloxycarbonyl or carboxyl, or phenylamino,

$R^7$ denotes straight-chain or branched alkyl which has 1 to 4 C atoms and is optionally substituted by 1 to 3 substituents from the series comprising fluorine and amino, phenyl, or methylphenyl,

$R^8$ denotes methoxycarbonyl, trichloromethyl, trifluoromethyl or dichlorofluoromethyl,

$R^2$, $R^3$, $R^4$ and $R^5$ can be identical or different and represent hydrogen, methyl, ethyl, n- or i-propyl, and X represents hydrogen, halogen or nitro, and their pharmaceutically usable acid addition, alkyli metal and alkaline earth metal salts and hydrates, for the preparation of medicaments.